# EUROPÄISCHE PATENTSCHRIFT

(11) **EP 1 025 086 B1**
(45) Veröffentlichungstag und Bekanntmachung des Hinweises auf die Patenterteilung: **25.06.2003**
(21) Anmeldenummer: 98948982.8
(22) Anmeldetag: 16.09.1998
(51) Int. Cl.: C07D 213/81, A61K 31/495, C07D 271/06, C07D 295/22, C07D 295/20, C07D 333/34, C07D 215/36, C07D 311/74, C07D 333/38, C07D 213/18, C07D 261/10, C07D 333/70, C07D 317/68, C07D 413/12

(54) **BENZAMIDINDERIVATE ALS FAKTOR XA-INHIBITOREN**
BENZAMIDINE DERIVATIVES AS FACTOR XA INHIBITORS
DERIVES DE BENZAMIDINE UTILISES COMME INHIBITEURS DU FACTEUR Xa

(30) Priorität: 01.10.1997 DE 19743435
(43) Veröffentlichungstag der Anmeldung: 09.08.2000
(73) Patentinhaber: MERCK PATENT GmbH, 64293 Darmstadt (DE)
(72) Erfinder: DORSCH, Dieter, D-64372 Ober-Ramstadt (DE); JURASZYK, Horst, D-64342 Seeheim (DE); WURZIGER, Hanns, D-64291 Darmstadt (DE); BERNOTAT-DANIELOWSKI, Sabine, D-61231 Bad Nauheim (DE); MELZER, Guido, D-65719 Hofheim (DE)
(86) Internationale Anmeldenummer: EP9805898
(87) Internationale Veröffentlichungsnummer: WO99016751

(56) Entgegenhaltungen:
- EP-A- 0 540 051
- EP-A- 0 608 759
- WO-A-92/08709
- WO-A-93/22303
- ELDRED C D ET AL: "Orally Active Non-Peptide Fibrinogen Receptor (GpIIb/IIIa) Antagonists: Identification of 4-[4-[4-(Aminoimino methyl)phenyl]-1-piperazinyl]-1- piperidineacetic Acid as a Long-Acting, Broad-Spectrum Antithrombotic Agent" J. MED. CHEM. (JMCMAR,00222623);94; VOL.37 (23); PP.3882-5, XP000579663 Glaxo Group Research Ltd.;Department of Medicinal Chemistry; Ware / Hertfordshire; SG12 ODP; UK (GB)
- STUERZEBECHER J ET AL: "Synthesis and Structure-Activity Relationships of Potent Thrombin Inhibitors: Piperazides of 3-Amidinophenylalanine" J. MED. CHEM. (JMCMAR,00222623);97; VOL.40 (19); PP.3091-3099, XP002077904 Zentrum fuer Vaskulaere Biologie und Medizin;Klinikum der Friedrich-Schiller-Universitaet Jena; Erfurt; D-99089; Germany (DE)

## Beschreibung

Die Erfindung betrifft Verbindungen der Formel I 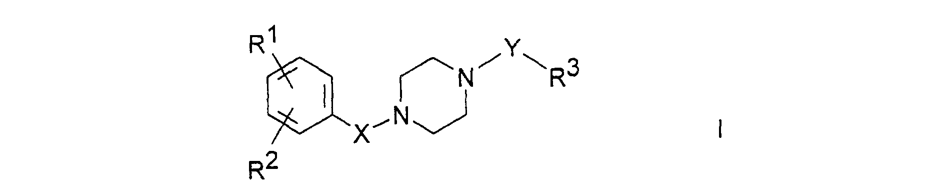 worin
- R¹: -C(=NH)-NH₂, das auch einfach durch -COA, -CO-[C(R⁶)₂]ₙ-Ar, -COOA, -OH oder durch eine konventionelle Aminoschutzgruppe substituiert sein kann,

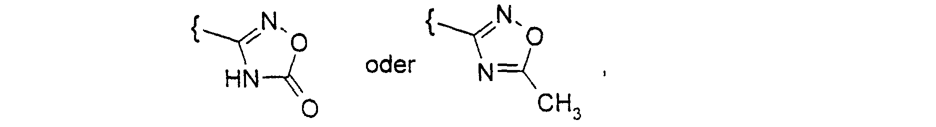

- R²: H, A, OR⁶, N(R⁶)₂, NO₂, CN, Hal, NHCOA, NHCOAr, NHSO₂A, NHSO₂Ar, COOR⁶, CON(R⁶)₂, CONHAr, COR⁶, COAr, S(O)ₙA oder S(O)ₙAr,
- R³: A, Cycloalkyl, -[C(R⁶)₂]ₙAr, -[C(R⁶)₂]ₙ-O-Ar, -[C(R⁶)₂]ₙHet oder -C(R⁶)₂=C(R⁶)₂-Ar,
- R⁶: H, A oder Benzyl,
- X: fehlt, -CO-, -C(R⁶)₂-, -C(R⁶)₂-C(R⁶)₂-, -C(R⁶)₂-CO-, -C(R⁶)₂-C(R⁶)₂-CO-, -C(R⁶)=C(R⁶)-CO-, NR⁶CO-, -N{[C(R⁶)₂]ₙ-COOR⁶}-CO- oder -C(COOR⁶)R⁶-C(R⁶)₂-CO-,
- Y: -C(R⁶)₂-, -SO₂-, -CO-, -COO- oder -CONR⁶-,
- A: Alkyl mit 1-20 C-Atomen, worin eine oder zwei CH₂-Gruppen durch O- oder S-Atome oder durch -CR⁶=CR⁶-Gruppen und/oder 1-7 H-Atome durch F ersetzt sein können,
- Ar: unsubstituiertes oder ein-, zwei- oder dreifach durch A, Ar', OR⁶, N(R⁶)₂, NO₂, CN, Hal, NHCOA, NHCOAr', NHSO₂A, NHSO₂Ar', COOR⁶, CON(R⁶)₂, CONHAr', COR⁶, COAr', S(O)ₙA oder S(O)ₙAr substituiertes Phenyl oder Naphthyl,
- Ar': unsubstituiertes oder ein-, zwei- oder dreifach durch A, OR⁶, N(R⁶)₂, NO₂, CN, Hal, NHCOA, COOR⁶, CON(R⁶)₂, COR⁶, oder S(O)ₙA substituiertes Phenyl oder Naphthyl,
- Het: ein- oder zweikerniges unsubstituiertes oder ein- oder mehrfach durch Hal, A, Ar', COOR⁶, CN, N(R⁶)₂, NO₂, Ar-CONH-CH₂ und/oder Carbonylsauerstoff substituiertes gesättigtes oder ungesättigtes heterocyclisches Ringsystem, welches eines, zwei, drei oder vier gleiche oder verschiedene Heteroatome wie Stickstoff, Sauerstoff und Schwefel enthält,
- Hal: F, Cl, Br oder I,
- n: 0, oder 2 bedeutet,
sowie deren Salze.

Gegenstand der Erfindung sind auch die optisch aktiven Formen, die Racemate, die Diastereomeren sowie die Hydrate und Solvate dieser Verbindungen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie Faktor Xa inhibierende Eigenschaften und können daher zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens eingesetzt werden.

Aromatische Amidinderivate mit antithrombotischer Wirkung sind z.B. aus der EP 0 540 051 B1 bekannt. Cyclische Guanidine zur Behandlung thromboembolischer Erkrankungen sind z.B. in der WO 97/08165 beschrieben. Aromatische Heterocyclen mit Faktor Xa inhibitorischer Aktivität sind z.B. aus der WO 96/10022 bekannt.

Aus EP 0 608 759 A2 sind strukturell verwandte Piperazinderivate als Hemmer des Fibrinogen rezeptors bekannt. Piperazide von 3-Amidinophenylalaninen als Thrombininhibitoren werden von J. Stuerzeberger et al. in J. Med. Chem. 40(19) (1997) 3091-3099 beschrieben und WO 92/08709 offenbart meta-substituierte Phenylalanin derivate als Thrombin - und Faktor Xa-Inhibitoren.

Der antithrombotische und antikoagulierende Effekt der erfindungsgemäßen Verbindungen wird auf die inhibierende Wirkung gegenüber der aktivierten Gerinnungsprotease, bekannt unter dem Namen Faktor Xa, zurückgeführt.
Faktor Xa ist eine der Proteasen, die in den komplexen Vorgang der Blutgerinnung involviert ist. Faktor Xa katalysiert die Umwandlung von Prothrombin in Thrombin, das seinerseits zur Thrombusbildung beiträgt. Eine Aktivierung von Thrombin kann zum Auftreten von thromboembolischen Erkrankungen führen.
Eine Inhibierung des Faktors Xa kann somit verhindern, daß Thrombin gebildet wird.
Die erfindungsgmäßen Verbindungen der Formel I sowie ihre Salze greifen durch Inhibierung des Faktors Xa in den Blutgerinnungsprozeß ein und hemmen so die Entstehung von Thromben.

Die Inhibierung des Faktors Xa durch die erfindungsgemäßen Verbindungen und die Messung der antikoagulierenden und antithrombotischen Aktivität kann nach üblichen in vitro- oder in vivo-Methoden ermittelt werden.

Ein geeignetes Verfahren wird z.B. von J. Hauptmann et al. in *Thrombosis and Haemostasis 63, 220-223 (1990)* beschrieben.

Die Messung der Inhibierung von Faktor Xa kann z.B. nach der Methode von T. Hara et al. in *Thromb. Haemostas. 71, 314-319 (1994)* erfolgen.

Die Verbindungen der Formel I können als Arzneimittelwirkstoffe in der Human- und Veterinärmedizin eingesetzt werden, insbesondere zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens.

Gegenstand der Erfindung sind die Verbindungen der Formel I und ihre Salze sowie ein Verfahren zur Herstellung von Verbindungen der Formel I nach Anspruch 1 sowie ihrer Salze, dadurch gekennzeichnet, daß man
a) sie aus einem ihrer funktionellen Derivate durch Behandeln mit einem solvolysierenden oder hydrogenolysierenden Mittel in Freiheit setzt, indem man
   i) eine Amidinogruppe aus ihrem Oxadiazolderivat durch Hydrogenolyse freisetzt,
   ii) eine konventionelle Aminoschutzgruppe durch Behandeln mit einem solvolysierenden oder hydrogenolysierenden Mittel durch Wasserstoff ersetzt oder
      eine durch eine konventionelle Schutzgruppe geschützte Aminogruppe in Freiheit setzt,
   oder
b) daß man zur Herstellung von Verbindungen der Formel I,
   worin R¹ 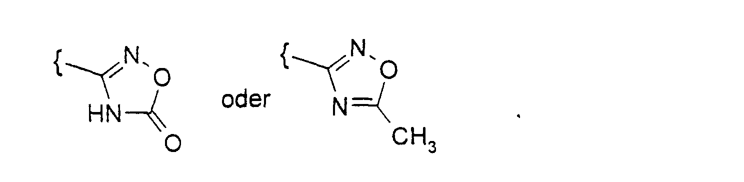
   X -CO- oder -C(R⁶)₂-CO-,
   und R², R³ und Y die in Anspruch 1 angegebenen Bedeutungen haben,
   eine Verbindung der Formel II 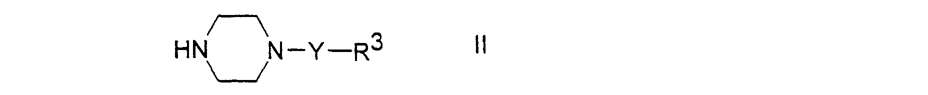 worin
   R³, R⁴, R⁵, W und Y die in Anspruch 1 angegebenen Bedeutungen haben,
   mit einer Verbindung der Formel III 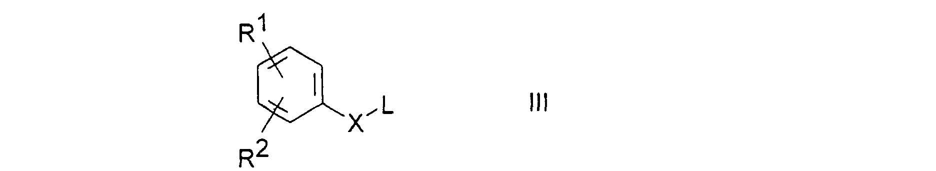 worin R¹ 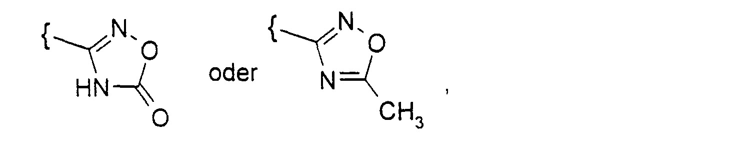
   X -CO- oder -C(R⁶)₂-CO- bedeutet,
   R² die in Anspruch 1 angegebene Bedeutung hat,
   und L Cl, Br, I oder eine freie oder reaktionsfähig funktionell abgewandelte OH-Gruppe bedeutet,
   umsetzt,
   oder
c) daß man zur Herstellung von Verbindungen der Formel I,
   worin R¹ 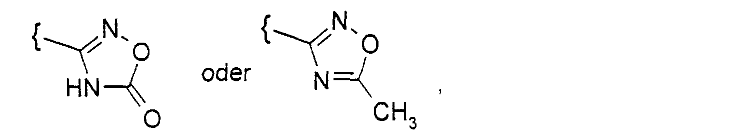
   Y -SO₂-, -CO-, -COO- oder -C(R⁶)₂- bedeutet,
   und R² und X die in Anspruch 1 angegebenen Bedeutungen haben,
   eine Verbindung der Formel IV

   L-Y-R³ IV

   worin
   Y -SO₂-, -CO-, -COO- oder -C(R⁶)₂- bedeutet,
   R³ die in Anspruch 1 angegebene Bedeutung hat, und L Cl, Br, I oder eine freie oder reaktionsfähig funktionell abgewandelte OH-Gruppe bedeutet,
   mit einer Verbindung der Formel V 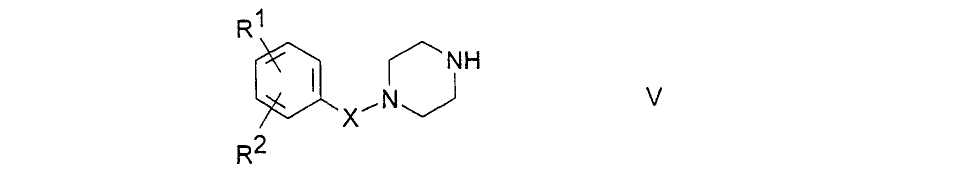 worin R¹ 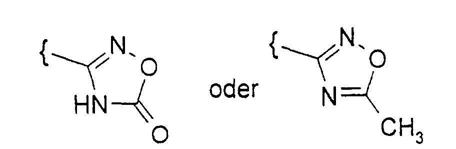 bedeutet, und R² und X die in Anspruch 1 angegebenen Bedeutungen haben, umsetzt,
   oder
d) daß man zur Herstellung von Verbindungen der Formel I,
   worin R¹ 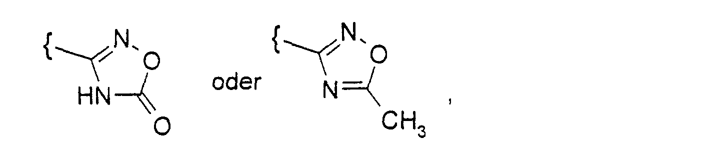
   Y -CONH- bedeutet,
   und R² und X die in Anspruch 1 angegebenen Bedeutungen haben,
   eine Verbindung der Formel VI

   R³-N=C=O VI

   worin R³ die in Anspruch 1 angegebene Bedeutung hat,
   mit einer Verbindung der Formel V 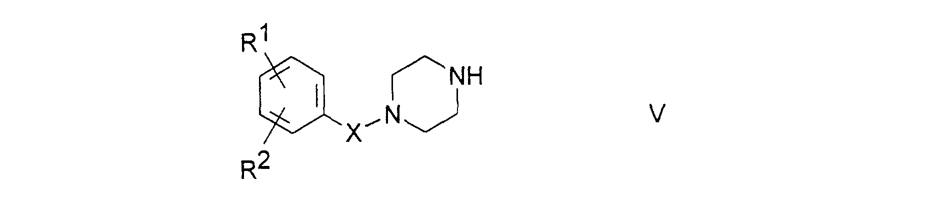 worin R¹ 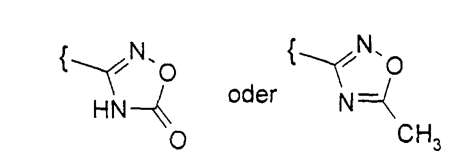 bedeutet, und R² und X die in Anspruch 1 angegebenen Bedeutungen haben,
   umsetzt,
   oder
e) daß man zur Herstellung von Verbindungen der Formel I,
   worin R¹ -C(=NH)-NH₂ bedeutet,
   eine Cyangruppe in eine Amidinogruppe umwandelt,
f) und/oder daß man in einer Verbindung der Formel I einen oder mehrere Rest(e) R¹, R² und/oder R³ in einen oder mehrere Rest(e) R¹, R² und/oder R³ umwandelt,
   indem man beispielsweise
   i) eine Estergruppe zu einer Carboxygruppe hydrolysiert,
   ii) eine Nitrogruppe reduziert,
   iii) eine Aminogruppe acyliert,
g) und/oder eine Base oder Säure der Formel I in eines ihrer Salze umwandelt.

Für alle Reste, die mehrfach auftreten, wie z.B. R⁶, gilt, daß deren Bedeutungen unabhängig voneinander sind.

Vor- und nachstehend haben die Reste bzw. Parameter L, X, Y, R¹, R² und R³ die bei den Formeln I bis VI angegebenen Bedeutungen, falls nicht ausdrücklich etwas anderes angegeben ist.

In den vorstehenden Formeln bedeutet A Alkyl und hat 1 bis 20, vorzugsweise 1, 2, 3, 4, 5, 6, 7, 8, 9, 10, 11 oder 12 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 12,2-Trimethylpropyl, Heptyl, Octyl, Nonyl oder Decyl.
Alkyl bedeutet weiterhin z.B. Trifluormethyl, Pentafluorethyl, Allyl oder Crotyl.

Cycloalkyl bedeutet vorzugsweise Cyclopropyl, Cyclobutyl, Cylopentyl, Cyclohexyl oder Cycloheptyl. Cycloalkyl bedeutet insbesondere den Rest eines bicyclischen Terpens, wie z.B. 3-Menthyl, ganz besonders bevorzugt ist der Campher-10-yl-Rest.

COR⁶ ist Acyl und bedeutet vorzugsweise Formyl, Acetyl, Propionyl, ferner auch Butyryl, Pentanoyl oder Hexanoyl.

Hal bedeutet vorzugsweise F, Cl oder Br, aber auch I.

R² bedeutet vorzugsweise H, Fluor, Chlor, Brom, lod, Hydroxy, Methoxy, Ethoxy, Propoxy, Nitro, Amino, Methylamino, Dimethylamino, Ethylamino, Diethylamino, Acetamido, Sulfonamido, Methylsulfonamido, Phenylsulfonamido, Methylthio, Ethylthio, Methylsulfinyl, Ethylsulfinyl, Methylsulfonyl, Ethylsulfonyl, Phenylsulfinyl, Phenylsulfonyl, Cyan, Carboxy, Methoxycarbonyl, Ethoxycarbonyl, ferner auch Acyl oder Benzoyl.

R³ bedeutet vorzugsweise z.B. A, Cycloalkyl, Ar, CH₂Ar, CH₂OAr, CH₂CH₂Ar, CH₂Het, CH₂CH₂Het oder CH=CH-Ar.

R° bedeutet H, A oder Benzyl, insbesondere jedoch H.

X bedeutet vorzugsweise z.B. fehlt, -CO-, -CH₂-, -CH₂-CH₂-, -CH₂-CO-, -CH₂-CH₂-CO-, -CH=CH-CO-, NR⁶CO-, -N{[CH₂]ₙ-COOR⁶}-CO- oder -CH(COOR⁶)-CH₂-CO-.

Y bedeutet vorzugsweise z.B. -SO₂- oder -CO-, ferner auch -COO-, -CONH- oder -CH₂-.

Ar bedeutet vorzugsweise unsubstituiertes Phenyl oder Naphthyl, weiterhin vorzugsweise z.B. durch A, Fluor, Chlor, Brom, lod, Hydroxy, Methoxy, Ethoxy, Propoxy, Butoxy, Pentyloxy, Hexyloxy, Benzyloxy, Phenethyloxy, Methylthio, Ethylthio, Methylsulfinyl, Ethylsulfinyl, Methylsulfonyl, Ethylsulfonyl, Phenylsulfinyl, Phenylsulfonyl, Nitro, Amino, Methylamino, Ethylamino, Dimethylamino, Diethylamino, Formamido, Acetamido, Propionylamino, Butyrylamino, Methylsulfonamido, Ethylsulfonamido, Propylsulfonamido, Butylsulfonamido, Phenylsulfonamido, (4-Methylphenyl)-sulfonamido, Carboxymethoxy, Carboxyethoxy, Methoxycarbonylmethoxy, Methoxycarbonylethoxy, Hydroxymethoxy, Hydroxyethoxy, Methoxyethoxy, Carboxy, Methoxycarbonyl, Ethoxycarbonyl, Cyan, Phenylaminocarbonyl, Acyl oder Benzoyl mono-, di- oder trisubstituiertes Phenyl oder Naphthyl, ferner auch Biphenyl.

Ar bedeutet daher bevorzugt z.B. o-, m- oder p-Tolyl, o-, m- oder p-Ethylphenyl, o-, m- oder p-Propylphenyl, o-, m- oder p-lsopropylphenyl, o-, m-oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-Acetamidophenyl, o-, m- oder p-Methoxyphenyl, o-, m- oder p-Ethoxyphenyl, o-, m- oder p-Carboxyphenyl, o-, m- oder p-Methoxycarbonylphenyl, o-, m- oder p-(N,N-Dimethylamino)-phenyl, o-, m- oder p-(N-Ethylamino)-phenyl, o-, m- oder p-(N,N-Diethylamino)-phenyl, o-, m- oder p-Acetylphenyl, o-, m- oder p-Formylphenyl, o-, m- oder p-Fluorphenyl, o-, m- oder p-Bromphenyl, o-, m- oder p- Chlorphenyl, o-, m- oder p-Methylsulfonylphenyl, o-, m- oder p-(Phenylsulfonamido)-phenyl, o-, m- oder p-(Methylsulfonamido)-phenyl, o-, m- oder p-Methylthiophenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet ganz besonders bevorzugt unsubstituiertes Phenyl oder Naphthyl, weiterhin vorzugsweise z.B. durch A, Chlor, Methoxy, Amino, Dimethylamino mono-, di- oder trisubstituiertes Phenyl oder Naphthyl, ferner auch Biphenyl.

Ar' bedeutet insbesondere z.B. Phenyl oder Naphthyl, ferner bevorzugt z.B. o-, m- oder p-Tolyl, o-, m- oder p-Ethylphenyl, o-, m- oder p-Propylphenyl, o-, m- oder p-lsopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-Acetamidophenyl, o-, m- oder p-Methoxyphenyl, o-, m- oder p-Ethoxyphenyl, o-, m-oder p-Carboxyphenyl, o-, m- oder p-Methoxycarbonylphenyl, o-, m- oder p-(N,N-Dimethylamino)-phenyl, o-, m- oder p-(N-Ethylamino)-phenyl, o-, m-oder p-(N,N-Diethylamino)-phenyl, o-, m- oder p-Acetylphenyl, o-, m- oder p-Formylphenyl, o-, m- oder p-Fluorphenyl, o-, m- oder p-Bromphenyl, o-, m- oder p- Chlorphenyl oder o-, m- oder p-Methylsulfonylphenyl.

Het bedeutet vorzugsweise z.B. 2- oder 3-Furyl, 2- oder 3-Thienyl, 1-, 2-oder 3-Pyrrolyl, 1-, 2, 4- oder 5-Imidazolyl, 1-, 3-, 4- oder 5-Pyrazolyl, 2-, 4-oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1-oder 5-Tetrazolyl, 1,2,3-Oxadiazol-4- oder -5-yl, 1,2,4-Oxadiazol-3- oder-5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6-oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4-oder -5-yl, 2,1,3-Benzoxadiazol-5-yl oder Dibenzofuranyl.

Die heterocyclischen Reste können auch teilweise oder vollständig hydriert sein.
Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder-4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder-4-pyranyl, 1,4-Dioxanyl, 1,3-Dioxan-2-, -4- oder -5-yl, Hexahydro-1-, -3-oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het ist unsubstituiert oder ein- oder mehrfach durch Hal, A, Ar', COOR⁶, CN, N(R⁶)₂, NO₂, Ar-CONH-CH₂ substituiert. "Mehrfach" bedeutet zwei-, drei-, vier- oder fünffach.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis If ausgedrückt werden, die der Formel I entsprechen und worin die nicht näher bezeichneten Reste die bei der Formel I angegebene Bedeutung haben, worin jedoch
- in la R¹: -C(=NH)-NH₂, das auch einfach durch -COA, -CO-[C(R⁶)₂]ₙ-Ar, -COOA, -OH oder durch eine konventionelle Aminoschutzgruppe substituiert sein kann, 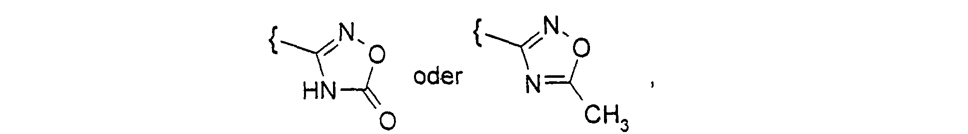
- R²: H, A, OR⁶, N(R⁶)₂, NO₂, CN, Hal, NHCOA, NHCOAr, NHSO₂A, NHSO₂Ar, COOR⁶, CON(R⁶)₂, CONHAr, COR⁶, COAr, S(O)ₙA oder S(O)ₙAr,
- R³: A, Cycloalkyl, Ar, CH₂Ar, CH₂OAr, CH₂CH₂Ar, CH₂Het, CH₂CH₂Het oder CH=CH-Ar,
- R⁶: H oder A,
- X: fehlt, -CO-, -CH₂-CO-, -CH₂-CH₂-CO-, -CH₂-, -CH₂-CH₂-, -CH=CH-CO-, -NHCO-, -N(CH₂COOR⁶)-CO- oder -CH(COOR⁶)-CH₂-CO-,
- Y: -SO₂-, -CO-, -COO-, -CO-NH- oder -CH₂-,
- A: Alkyl mit 1-20 C-Atomen, worin eine oder zwei CH₂- Gruppen durch O- oder S-Atome oder durch -CR⁶=CR⁶-Gruppen und/oder 1-7 H-Atome durch F ersetzt sein können,
- Ar: unsubstituiertes oder ein-, zwei- oder dreifach durch A, Ar', OR⁶, N(R⁶)₂, NO₂, CN, Hal, NHCOA, NHCOAr', NHSO₂A, NHSO₂Ar', COOR⁶, CON(R⁶)₂, CONHAr', COR⁶, COAr', S(O)ₙA oder S(O)ₙAr substituiertes Phenyl oder Naphthyl,
- Ar': unsubstituiertes oder ein-, zwei- oder dreifach durch A, OR⁶, N(R⁶)₂, NO₂, CN, Hal, NHCOA, COOR⁶, CON(R⁶)₂, COR⁶, oder S(O)ₙA substituiertes Phenyl oder Naphthyl,
- Het: ein- oder zweikerniges unsubstituiertes oder ein- oder mehrfach durch Hal, A, Ar', COOR⁶, CN, N(R⁶)₂, NO₂, Ar-CONH-CH₂ und/oder Carbonylsauerstoff substituiertes gesättigtes oder ungesättigtes heterocyclisches Ringsystem, welches eines, zwei, drei oder vier gleiche oder verschiedene Heteroatome wie Stickstoff, Sauerstoff und Schwefel enthält,
- Hal: F, Cl, Br oder I und
- n: 0, 1 oder 2
bedeutet;
- in lb R¹: -C(=NH)-NH₂, das auch einfach durch -COA, -CO-[C(R⁶)₂]ₙ-Ar, -COOA, -OH oder durch eine konventionelle Aminoschutzgruppe substituiert sein kann, 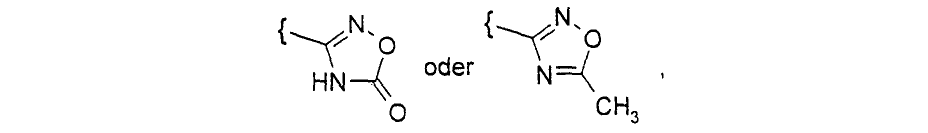
- R²: H,
- R³: A, Cycloalkyl, Ar, -CH₂Ar, -CH₂OAr, -CH₂CH₂Ar, -CH₂Het, -CH₂CH₂Het oder -CH=CH-Ar,
- R⁶: H oder A,
- X: fehlt, -CO-, -CH₂-CO-, -CH₂-CH₂-CO-, -CH₂-, -CH₂-CH₂-, -CH=CH-CO-, -NHCO-, -N(CH₂COOR⁶)-CO- oder -CH(COOR⁶)-CH₂-CO-,
- Y: -SO₂-, -CO-, -COO-, -CO-NH- oder -CH₂-,
- A: Alkyl mit 1-20 C-Atomen, worin eine oder zwei CH₂- Gruppen durch O- oder S-Atome oder durch -CR⁶=CR⁶-Gruppen und/oder 1-7 H-Atome durch F ersetzt sein können,
- Ar: unsubstituiertes oder ein-, zwei- oder dreifach durch A, Ar', OR⁶, NH₂, NO₂, CN, Hal, NHCOA, NHCOAr', NHSO₂A, NHSO₂Ar', COOR⁶, CON(R⁶)₂, CONHAr', COR⁶, COAr', S(O)ₙA oder S(O)ₙAr substituiertes Phenyl oder Naphthyl,
- Ar': unsubstituiertes oder ein-, zwei- oder dreifach durch A, OR⁶, N(R⁶)₂, NO₂, CN, Hal, NHCOA, COOR⁶, CON(R⁶)₂, COR⁶, oder S(O)ₙA substituiertes Phenyl oder Naphthyl,
- Het: ein- oder zweikerniges unsubstituiertes oder ein- oder mehrfach durch Hal, A, Ar', COOR⁶, CN, N(R⁶)₂, NO₂, Ar-CONH-CH₂ und/oder Carbonylsauerstoff substituiertes gesättigtes oder ungesättigtes heterocyclisches Ringsystem, welches eines, zwei, drei oder vier gleiche oder verschiedene Heteroatome wie Stickstoff, Sauerstoff und Schwefel enthält,
- Hal: F, Cl, Br oder I und
- n: 0, 1 oder 2
bedeutet;
- in Ic R¹: -C(=NH)-NH₂, das auch einfach durch -COA, -CO-[C(R⁶)₂]ₙ-Ar, -COOA, -OH oder durch eine konventionelle Aminoschutzgruppe substituiert sein kann, 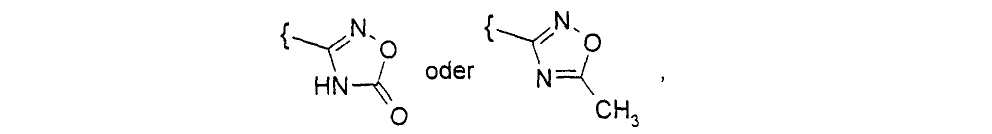
- R²: H,
- R³: A, Cycloalkyl, Ar, -CH₂Ar, -CH₂OAr, -CH₂CH₂Ar, -CH₂Het, -CH₂CH₂Het oder -CH=CH-Ar,
- R⁶: H oder A,
- X: fehlt, -CO-, -CH₂-CO-, -CH₂-CH₂-CO-, -CH₂-, -CH₂-CH₂-, -CH=CH-CO-, -NHCO-, -N{CH₂-COOR⁶}-CO- oder -CH(COOR⁶)-CH₂-CO-,
- Y: -SO₂-, -CO-, -COO-, -CO-NH- oder -CH₂-,
- A: Alkyl mit 1-20 C-Atomen, worin eine oder zwei CH₂- Gruppen durch O- oder S-Atome oder durch -CR⁶=CR⁶-Gruppen und/oder 1-7 H-Atome durch F ersetzt sein können,
- Ar: unsubstituiertes oder ein-, zwei- oder dreifach durch A, Ar', OR⁶, N(R⁶)₂, NO₂, CN, Hal, NHCOA, NHCOAr', NHSO₂A, NHSO₂Ar', COOR⁶, CON(R⁶)₂, CONHAr', COR⁶, COAr', S(O)ₙA oder S(O)ₙAr substituiertes Phenyl oder Naphthyl,
- Ar': unsubstituiertes oder ein-, zwei- oder dreifach durch A, OR⁶, N(R⁶)₂, NO₂, CN, Hal, NHCOA, COOR⁶, CON(R⁶)₂, COR⁶, oder S(O)ₙA substituiertes Phenyl oder Naphthyl,
- Het: ein- oder zweikerniger unsubstituierter oder ein- oder mehrfach durch Hal, A, Ar', COOR⁶, CN, N(R⁶)₂, NO₂, Ar-CONH-CH₂ und/oder Carbonylsauerstoff substituiertes heterocyclisches Ringsystem ausgewählt aus der Gruppe
Thiophen, Tetrahydrochinolin, Chroman, Pyrazol, Isoxazol, Pyridin, Benzodioxol oder Benzothiophen,
- Hal: F, Cl, Br oder l und
- n: 0, 1 oder 2
bedeutet;
- in Id R¹: -C(=NH)-NH₂, das auch einfach durch -COA, -CO-[C(R⁶)₂]ₙ-Ar, -COOA, -OH oder durch eine konventionelle Aminoschutzgruppe substituiert sein kann, 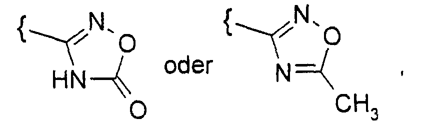
- R²: H,
- R³: A, Cycloalkyl, Ar, -CH₂Ar, -CH₂OAr, -CH₂CH₂Ar, -CH₂Het, -CH₂CH₂Het oder -CH=CH-Ar,
- R⁶: H oder A,
- X: fehlt, -CO-, -CH₂-CO-, -CH₂-CH₂-CO-, -CH₂-, -CH₂-CH₂-, -CH=CH-CO-, -NHCO-, -N{CH₂-COOR⁶}-CO- oder -CH(COOR⁶)-CH₂-CO-,
- Y: -SO₂-, -CO-, -CO-NH- oder -CH₂-,
- A: Alkyl mit 1-20 C-Atomen, worin eine oder zwei CH₂- Gruppen durch O- oder S-Atome oder durch -CR⁶=CR⁶-Gruppen und/oder 1-7 H-Atome durch F ersetzt sein können,
- Ar: unsubstituiertes oder ein-, zwei- oder dreifach durch A, Ar', OR⁶, N(R⁶)₂, NO₂, CN, Hal, NHCOA, NHCOAr', NHSO₂A, NHSO₂Ar', COOR⁶, CON(R⁶)₂, CONHAr', COR⁶, COAr', S(O)ₙA oder S(O)ₙAr substituiertes Phenyl oder Naphthyl,
- Ar': Phenyl,
- Het: ein- oder zweikerniger unsubstituierter oder ein- oder mehrfach durch Hal, A, Ar', COOR⁶, CN, N(R⁶)₂, NO₂, Ar-CONH-CH₂ und/oder Carbonylsauerstoff substituiertes heterocyclisches Ringsystem ausgewählt aus der Gruppe
Thiophen, Tetrahydrochinolin, Chroman, Pyrazol, Isoxazol, Pyridin, Benzodioxol, Benzothiophen oder Dibenzofuran,
- Hal: F, Cl, Br oder I und
- n: 0, 1 oder 2
bedeutet;
- in le R¹: -C(=NH)-NH₂, das auch einfach durch -COA, -CO-[C(R⁶)₂]ₙ-Ar, -COOA, -OH oder durch eine konventionelle Aminoschutzgruppe substituiert sein kann, 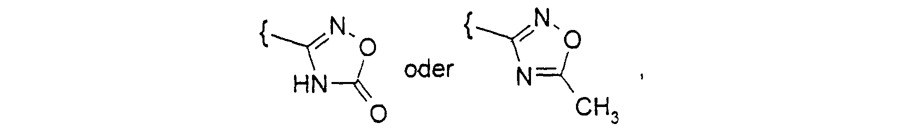
- R²: H,
- R³: A, Cycloalkyl, Ar, -CH₂Ar, -CH₂OAr, -CH₂CH₂Ar, -CH₂Het, -CH₂CH₂Het oder -CH=CH-Ar,
- R⁶: H oder A,
- X: fehlt, -CO-, -CH₂-CO-, -CH₂-CH₂-CO-, -CH₂-, -CH₂-CH₂-, -NHCO-, -N{CH₂-COOR⁶}-CO- oder -CH(COOR⁶)-CH₂-CO-,
- Y: -SO₂-, -CO- oder -CH₂-,
- A: Alkyl mit 1-20 C-Atomen, worin eine oder zwei CH₂- Gruppen durch O- oder S-Atome oder durch -CR⁶=CR⁶-Gruppen und/oder 1-7 H-Atome durch F ersetzt sein können,
- Ar: unsubstituiertes oder ein-, zwei- oder dreifach durch A, Ar', OR⁶, N(R⁶)₂, NO₂, CN, Hal, NHCOA, NHCOAr', NHSO₂A, NHSO₂Ar', COOR⁶, CON(R⁶)₂, CONHAr', COR⁶, COAr', S(O)ₙA oder S(O)ₙAr substituiertes Phenyl oder Naphthyl,
- Ar': Phenyl,
- Het: ein- oder zweikerniger unsubstituierter oder ein- oder mehrfach durch Hal, A, Ar', COOR⁶, CN, N(R⁶)₂, NO₂, Ar-CONH-CH₂ und/oder Carbonylsauerstoff substituiertes heterocyclisches Ringsystem ausgewählt aus der Gruppe
Thiophen, Tetrahydrochinolin, Chroman, Pyrazol, Isoxazol, Pyridin, Benzodioxol, Benzothiophen oder Dibenzofuran,
- Hal: F, Cl, Br oder I und
- n: 0, 1 oder 2
bedeutet;
- in If R¹: -C(=NH)-NH₂, das auch einfach durch COOA substituiert sein kann, 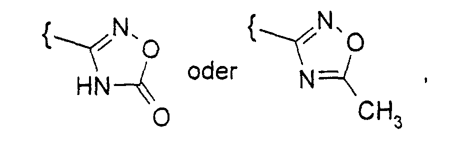
- R²: H,
- R³: A, Cycloalkyl, Ar, -CH₂Ar, -CH₂OAr, -CH₂CH₂Ar, -CH₂Het, -CH₂CH₂Het oder -CH=CH-Ar,
- R⁶: H oder A,
- X: fehlt, -CO-, -CH₂-CO-, -CH₂-CH₂-CO-, -CH₂-, -CH₂-CH₂-, -NHCO-, -N{CH₂-COOR⁶}-CO- oder -CH(COOR⁶)-CH₂-CO-,
- Y: -SO₂-, -CO- oder -CH₂-,
- A: Alkyl mit 1-20 C-Atomen, worin eine oder zwei CH₂- Gruppen durch O- oder S-Atome oder durch -CR⁶=CR⁶-Gruppen und/oder 1-7 H-Atome durch F ersetzt sein können,
- Ar: unsubstituiertes oder ein-, zwei- oder dreifach durch A, Ar', OR⁶, N(R⁶)₂, NO₂, CN, Hal, NHCOA, NHCOAr', NHSO₂A, NHSO₂Ar', COOR⁶, CON(R⁶)₂, CONHAr', COR⁶, COAr', S(O)ₙA oder S(O)ₙAr substituiertes Phenyl oder Naphthyl,
- Ar': Phenyl,
- Het: ein- oder zweikerniger unsubstituierter oder ein- oder mehrfach durch Hal, A, Ar', COOR⁶, CN, N(R⁶)₂, NO₂, Ar-CONH-CH₂ und/oder Carbonylsauerstoff substituiertes heterocyclisches Ringsystem ausgewählt aus der Gruppe
Thiophen, Tetrahydrochinolin, Chroman, Pyrazol, Isoxazol, Pyridin, Benzodioxol, Benzothiophen oder Dibenzofuran,
- Hal: F, Cl, Br oder I und
- n: 0, 1 oder 2
bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Verbindungen der Formel I können vorzugsweise erhalten werden, indem man Verbindungen der Formel I aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.
Bevorzugte Ausgangsstoffe sind auch die Oxadiazolderivate, die in die entsprechenden Amidinoverbindungen überführt werden können.

Die Einführung der Oxadiazolgruppe gelingt z.B. durch Umsetzung der Cyanverbindungen mit Hydroxylamin und Reaktion mit Phosgen, Dialkylcarbonat, Chlorameisensäureester, N,N'-Carbonyldiimidazol oder Acetanhydrid.

Es können auch mehrere - gleiche oder verschiedene - geschützte Aminound/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyloder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCI in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ, Benzyl oder die Freisetzung der Amidinogruppe aus ihrem Oxadiazolderivat)) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Verbindungen der Formel I,
worin R¹ 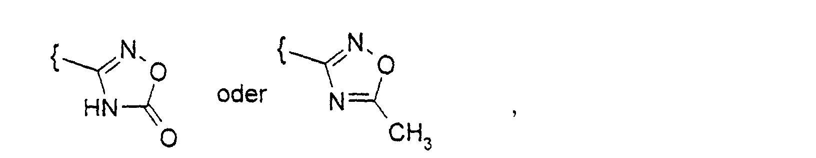
X -CO- oder -C(R⁶)₂-CO-,
und R², R³ und Y die in Anspruch 1 angegebenen Bedeutungen haben, können vorzugsweise erhalten werden, indem man Verbindungen der Formel II mit Verbindungen der Formel III umsetzt.

In den Verbindungen der Formel III bedeutet L vorzugsweise Cl, Br, I oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin oder eines Überschusses der Aminkomponente der Formel II bzw. des Alkylierungsderivates der Formel III kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Ausgangsverbindungen der Formel II und III sind in der Regel bekannt. Sind sie neu, so können aber nach an sich bekannten Methoden hergestellt werden.

Verbindungen der Formel I,
worin R¹ 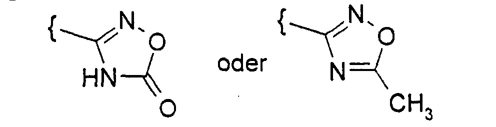 bedeutet,
Y SO₂, CO oder COO bedeutet,
und R² und X die in Anspruch 1 angegebenen Bedeutungen haben, können vorzugsweise erhalten werden, indem man Verbindungen der Formel IV mit Verbindungen der Formel V umsetzt.

In den Verbindungen der Formel IV bedeutet L vorzugsweise Cl, Br, I oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung der Verbindungen der Formel IV mit Verbindungen der Formel V erfolgt vorzugsweise in einem inerten Lösungsmittel, unter Zusatz einer Base und bei Temperaturen wie oben angegeben.

Die Ausgangsverbindungen der Formel IV und V sind in der Regel bekannt. Sind sie neu, so können aber nach an sich bekannten Methoden hergestellt werden.

Verbindungen der Formel I,
worin R¹ 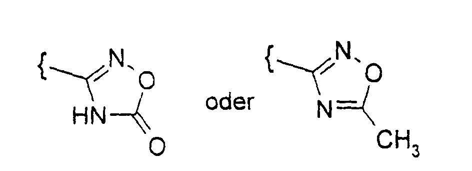 bedeutet,
Y CONH bedeutet,
und R² und X die in Anspruch 1 angegebenen Bedeutungen haben, können vorzugsweise erhalten werden, indem man Verbindungen der Formel VI mit Verbindungen der Formel V umsetzt.
Die Umsetzung der Verbindungen der Formel VI mit Verbindungen der Formel V erfolgt vorzugsweise in einem inerten Lösungsmittel und bei Temperaturen wie oben angegeben.

Die Ausgangsverbindungen der Formel VI sind in der Regel bekannt. Sind sie neu, so können aber nach an sich bekannten Methoden hergestellt werden.

Verbindungen der Formel I, worin R¹ -C(=NH)-NH₂ bedeutet, können ferner aus der entsprechenden Cyanverbindung erhalten werden.
Die Umwandlung einer Cyangruppe in eine Amidinogruppe erfolgt durch Umsetzung mit z.B. Hydroxylamin und anschließender Reduktion des N-Hydroxyamidins mit Wasserstoff in Anwesenheit eines Katalysators wie z.B. Pd/C.
Zur Herstellung eines Amidins der Formel I (R¹ = -C(=NH)-NH₂) kann man an ein Nitril der Formel I (R¹ = CN) auch Ammoniak anlagern. Die Anlagerung erfolgt bevorzugt mehrstufig, indem man in an sich bekannter Weise a) das Nitril mit H₂S in ein Thioamid umwandelt, das mit einem Alkylierungsmittel, z.B. CH₃I, in den entsprechenden S-Alkyl-imidothioester übergeführt wird, welcher seinerseits mit NH₃ zum Amidin reagiert, b) das Nitril mit einem Alkohol, z.B. Ethanol in Gegenwart von HCI in den entsprechenden Imidoester umwandelt und diesen mit Ammoniak behandelt, oder c) das Nitril mit Lithium-bis-(trimethylsilyl)-amid umsetzt und das Produkt anschließend hydrolysiert.

Es ist ferner möglich, eine Verbindung der Formel I in eine andere Verbindung der Formel I umzuwandeln, indem man einen oder mehrere Rest(e) R¹, R², R³, R⁴ und/oder R⁵ in einen oder mehrere Rest(e) -R¹, R², R³, R⁴ und/oder R⁵ umwandelt, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert.

Ester können z.B. mit Essigsäure oder mit NaOH oder KOH in Wasser, Wasser-THF oder Wasser-Dioxan bei Temperaturen zwischen 0 und 100° verseift werden.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Eine Base der Formel I kann mit einer Säure in das zugehörige Säureadditionssaiz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natriumoder Kaliumhydroxid oder -carbonat) in die entsprechenden Metall-, insbesondere Alkalimetall- oder Erdalkalimetall-, oder in die entsprechenden Ammoniumsalze umgewandelt werden.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens verwendet werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
Massenspektrometrie (MS): El (Elektronenstoß-lonisation) M⁺
FAB (Fast Atom Bombardment) (M+H)⁺

### Beispiel 1

Zu einer Lösung von 10,0 g 4-(5-Methyl-1,2,4-oxadiazol-3-yl)-benzoesäure in 150 ml Toluol gibt man 46 ml Thionylchlorid und 1 ml DMF. Die Lösung wird 5 Stunden unter Rückfluß erhitzt. Nach Entfernen des Lösungsmittels erhält man 4-(5-Methyl-1,2,4-oxadiazol-3-yl)-benzoylchlorid, El 222.
Durch anschließende Umsetzung mit 9,3 g 1-tert-Butoxycarbonylpiperazin in 150 ml Dichlormethan und 48 ml Triethylamin erhält nach üblicher Aufarbeitung 4-[4-(5-Methyl-[1,2,4]oxadiazol-3-yl)-benzoyl]-piperazin-1-carbonsäure-tert.-butylester, FAB 373.
Die Abspaltung der BOC-Gruppe erfolgt mit 4N HCI in Dioxan.
Eine Lösung von 100 mg des erhaltenen [4-(5-Methyl-[1,2,4]oxadiazol-3-yl)-phenyl]-piperazin-1-yl-methanon ("A") und 120 mg 6-Chlornaphthalin-2-sulfonylchlorid in 5 ml Dichlormethan wird mit 400 mg 4-Dimethylaminopyridin auf Polystyrol versetzt und 18 Stunden bei Raumtemperatur nachgerührt. Nach Filtration und Entfernen des Lösungsmittels erhält man [4-(6-Chlornaphthalin-2-sulfonyl)-piperazin-1-yl]-[4-(5-methyl-[1,2,4]oxadiazol-3-yl)-phenyl]-methanon, FAB 497.

Analog erhält man durch Umsetzung von "A"
mit 4-Biphenylyl-2-sulfonylchlorid
   [4-(4-Biphenylylsulfonyl)-piperazin-1-yl]-[4-(5-methyl-[1,2,4]oxadiazol-3-yl)-phenyl]-methanon;
mit 2-Naphthyl-sulfonylchlorid
   [4-(2-Naphthyl-sulfonyl)-piperazin-1-yl]-[4-(5-methyl-[1,2,4]oxadiazol-3-yl)-phenyl]-methanon;
mit 4-Propylphenyl-sulfonylchlorid
   [4-(4-Propylphenyl-sulfonyl)-piperazin-1-yl]-[4-(5-methyl-[1,2,4]oxadiazol-3-yl)-phenyl]-methanon;
mit 2-Phenylvinyl-sulfonylchlorid
   [4-(2-Phenylvinyl-sulfonyl)-piperazin-1-yl]-[4-(5-methyl-[1,2,4]oxadiazol-3-yl)-phenyl]-methanon;
mit 3-Nitro-4-chlorphenyl-sulfonylchlorid
   [4-(3-Nitro-4-chlorphenyl-sulfonyl)-piperazin-1-yl]-[4-(5-methyl-[1,2,4]oxadiazol-3-yl)-phenyl]-methanon;
mit 2-Nitro-4-methoxyphenyl-sulfonylchlorid
   [4-(2-Nitro-4-methoxyphenyl-sulfonyl)-piperazin-1-yl]-[4-(5-methyl-[1,2,4]oxadiazol-3-yl)-phenyl]-methanon;
mit p-Tolyl-sulfonylchlorid
   [4-(4-Tolylsulfonyl)-piperazin-1-yl]-[4-(5-methyl-[1,2,4]oxadiazol-3-yl)-phenyl]-methanon;
mit Decylsulfonylchlorid
   [4-(Decylsulfonyl)-piperazin-1-yl]-[4-(5-methyl-[1,2,4]oxadiazol-3-yl)-phenyl]-methanon;
mit Benzylsulfonylchlorid
   [4-(Benzylsulfonyl)-piperazin-1-yl]-[4-(5-methyl-[1,2,4]oxadiazol-3-yl)-phenyl]-methanon;
mit 3-Nitro-6-methylbenzyl-sulfonylchlorid
   [4-(3-Nitro-6-methylbenzyl-sulfonyl)-piperazin-1-yl]-[4-(5-methyl-[1,2,4]oxadiazol-3-yl)-phenyl]-methanon;
mit 2,3-Dichlorphenyl-sulfonylchlorid
   [4-(2,3-Dichlorphenyl-sulfonyl)-piperazin-1-yl]-[4-(5-methyl-[1,2,4]oxadiazol-3-yl)-phenyl]-methanon;
mit 3,4-Dichlorphenyl-sulfonylchlorid
   [4-(3,4-Dichlorphenyl-sulfonyl)-piperazin-1-yl]-[4-(5-methyl-[1,2,4]oxadiazol-3-yl)-phenyl]-methanon;
mit Phenylsulfonylchlorid
   [4-(Phenylsulfonyl)-piperazin-1-yl]-[4-(5-methyl-[1,2,4]oxadiazol-3-yl)-phenyl]-methanon;
mit 3-Bromphenyl-sulfonylchlorid
   [4-(3-Bromphenyl-sulfonyl)-piperazin-1-yl]-[4-(5-methyl-[1,2,4]oxadiazol-3-yl)-phenyl]-methanon;
mit 3,4-Dimethoxyphenyl-sulfonylchlorid
   [4-(3,4-Dimethoxyphenyl-sulfonyl)-piperazin-1-yl]-[4-(5-methyl-[1,2,4]oxadiazol-3-yl)-phenyl]-methanon;
mit 4-Acetamido-3-chlorphenyl-sulfonylchlorid
   [4-(4-Acetamido-3-chlorphenyl-sulfonyl)-piperazin-1-yl]-[4-(5-methyl-[1,2,4]oxadiazol-3-yl)-phenyl]-methanon;
mit 4-Chlor-2,5-dimethylphenyl-sulfonylchlorid
   [4-(4-Chlor-2,5-dimethylphenyl-sulfonyl)-piperazin-1-yl]-[4-(5-methyl-[1,2,4]oxadiazol-3-yl)-phenyl]-methanon;
mit m-Tolyl-sulfonylchlorid
   [4-(3-Tolylsulfonyl)-piperazin-1-yl]-[4-(5-methyl-[1,2,4]oxadiazol-3-yl)-phenyl]-methanon;
mit 2-Methoxy-5-methylphenyl-sulfonylchlorid
   [4-(2-Methoxy-5-methylphenyl-sulfonyl)-piperazin-1-yl]-[4-(5-methyl-[1,2,4]oxadiazol-3-yl)-phenyl]-methanon;
mit 3-Chlorphenyl-sulfonylchlorid
   [4-(3-Chlorphenyl-sulfonyl)-piperazin-1-yl]-[4-(5-methyl-[1,2,4]oxadiazol-3-yl)-phenyl]-methanon;
mit 4-Methoxyphenyl-sulfonylchlorid
   [4-(4-Methoxyphenyl-sulfonyl)-piperazin-1-yl]-[4-(5-methyl-[1,2,4]oxadiazol-3-yl)-phenyl]-methanon;
mit 2-Thienyl-sulfonylchlorid
   [4-(2-Thienyl-sulfonyl)-piperazin-1-yl]-[4-(5-methyl-[1,2,4]oxadiazol-3-yl)-phenyl]-methanon;
mit 4-Chlorphenyl-sulfonylchlorid
   [4-(4-Chlorphenyl-sulfonyl)-piperazin-1-yl]-[4-(5-methyl-[1,2,4]oxadiazol-3-yl)-phenyl]-methanon;
mit Isopropylsulfonylchlorid
   [4-(Isopropylsulfonyl)-piperazin-1-yl]-[4-(5-methyl-[1,2,4]oxadiazol-3-yl)-phenyl]-methanon;
mit 8-Chinolylsulfonylchlorid
   [4-(8-Chinolylsulfonyl)-piperazin-1-yl]-[4-(5-methyl-[1,2,4]oxadiazol-3-yl)-phenyl]-methanon;
mit 4-Nitro-phenyl-sulfonylchlorid
   [4-(4-Nitrophenyl-sulfonyl)-piperazin-1-yl]-[4-(5-methyl-[1,2,4]oxadiazol-3-yl)-phenyl]-methanon;
mit 3-Chlor-6-methoxyphenyl-sulfonylchlorid
   [4-(3-Chlor-6-methoxyphenyl-sulfonyl)-piperazin-1-yl]-[4-(5-methyl-[1,2,4]oxadiazol-3-yl)-phenyl]-methanon;
mit 4-Acetamidophenyl-sulfonylchlorid
   [4-(4-Acetamidophenyl-sulfonyl)-piperazin-1-yl]-[4-(5-methyl-[1,2,4]oxadiazol-3-yl)-phenyl]-methanon;
mit 2,2,5,7,8-Pentamethylchroman-6-yl-sulfonylchlorid
   [4-(2,2,5,7,8-Pentamethylchroman-6-yl-sulfonyl)-piperazin-1-yl]-[4-(5-methyl-[1,2,4]oxadiazol-3-yl)-phenyl]-methanon;
mit Campher-10-yl-sulfonylchlorid
   [4-(Campher-10-yl-sulfonyl)-piperazin-1-yl]-[4-(5-methyl-[1,2,4]oxadiazol-3-yl)-phenyl]-methanon;
mit 5-(1-Methyl-5-trifluormethyl-3-pyrazolyl)-2-thienyl-sulfonylchlorid
   {4-[5-(1-Methyl-5-trifluormethyl-3-pyrazolyl)-2-thienylsulfonyl]-piperazin-1-yl}-[4-(5-methyl-[1,2,4]oxadiazol-3-yl)-phenyl]-methanon;
mit 2,5-Dichlorphenyl-sulfonylchlorid
   [4-(2,5-Dichlorphenyl-sulfonyl)-piperazin-1-yl]-[4-(5-methyl-[1,2,4]oxadiazol-3-yl)-phenyl]-methanon;
mit 2,4,6-Trimethylphenyl-sulfonylchlorid
   [4-(2,4,6-Trimethylphenyl-sulfonyl)-piperazin-1-yl]-[4-(5-methyl-[1,2,4]oxadiazol-3-yl)-phenyl]-methanon;
mit 2-Methylsulfonylphenyl-sulfonylchlorid
   [4-(2-Methylsulfonylphenyl-sulfonyl)-piperazin-1-yl]-[4-(5-methyl-[1,2,4]oxadiazol-3-yl)-phenyl]-methanon;
mit 5-Benzamidomethyl-2-thienyl-sulfonylchlorid
   [4-(5-Benzamidomethyl-2-thienyl-sulfonyl)-piperazin-1-yl]-[4-(5-methyl-[1,2,4]oxadiazol-3-yl)-phenyl]-methanon;
mit Methylsulfonylchlorid
   [4-(Methylsulfonyl)-piperazin-1-yl]-[4-(5-methyl-[1,2,4]oxadiazol-3-yl)-phenyl]-methanon;
mit 1,3-Dimethyl-5-chlor-4-pyrazolyl-sulfonylchlorid
   [4-(1,3-Dimethyl-5-chlor-4-pyrazolyl-sulfonyl)-piperazin-1-yl]-[4-(5-methyl-[1,2,4]oxadiazol-3-yl)-phenyl]-methanon;
mit 3,5-Dimethyl-4-isoxazolyl-sulfonylchlorid
   [4-(3,5-Dimethyl-4-isoxazolyl-sulfonyl)-piperazin-1-yl]-[4-(5-methyl-[1,2,4]oxadiazol-3-yl)-phenyl]-methanon;
mit 4-Brom-2-ethylphenyl-sulfonylchlorid
   [4-(4-Brom-2-ethylphenyl-sulfonyl)-piperazin-1-yl]-[4-(5-methyl-[1,2,4]oxadiazol-3-yl)-phenyl]-methanon;
mit 1-Naphthylsulfonylchlorid
   [4-(1-Naphthylsulfonyl)-piperazin-1-yl]-[4-(5-methyl-[1,2,4]oxadiazol-3-yl)-phenyl]-methanon;
mit 5-Dimethylamino-1-naphthylsulfonylchlorid
   [4-(5-Dimethylamino-1-naphthylsulfonyl)-piperazin-1-yl]-[4-(5-methyl-[1,2,4]oxadiazol-3-yl)-phenyl]-methanon;
mit 3,4-Difluorphenyl-sulfonylchlorid
   [4-(3,4-Difluorphenyl-sulfonyl)-piperazin-1-yl]-[4-(5-methyl-[1,2,4]oxadiazol-3-yl)-phenyl]-methanon;
mit 4-tert.-Butylphenyl-sulfonylchlorid
   [4-(4-tert.-Butylphenyl-sulfonyl)-piperazin-1-yl]-[4-(5-methyl-[1,2,4]oxadiazol-3-yl)-phenyl]-methanon;
mit 4-Ethylphenyl-sulfonylchlorid
   [4-(4-Ethylphenyl-sulfonyl)-piperazin-1-yl]-[4-(5-methyl-[1,2,4]oxadiazol-3-yl)-phenyl]-methanon;
mit 4-(1,1-Dimethylpropyl)-phenyl-sulfonylchlorid
   [4-(4-(1,1-Dimethylpropyl)-phenyl-sulfonyl)-piperazin-1 -yl]-[4-(5-methyl-[1,2,4]oxadiazol-3-yl)-phenyl]-methanon;
mit 4-Isopropylphenyl-sulfonylchlorid
   [4-(4-lsopropylphenyl-sulfonyl)-piperazin-1-yl]-[4-(5-methyl-[1,2,4]oxadiazol-3-yl)-phenyl]-methanon;
mit 4-Trifluormethylphenyl-sulfonylchlorid
   [4-(4-Trifluormethylphenyl-sulfonyl)-piperazin-1-yl]-[4-(5-methyl-[1,2,4]oxadiazol-3-yl)-phenyl]-methanon;
mit 3-Nitro-4-methylphenyl-sulfonylchlorid
   [4-(3-Nitro-4-methylphenyl-sulfonyl)-piperazin-1-yl]-[4-(5-methyl-[1,2,4]oxadiazol-3-yl)-phenyl]-methanon;
mit 4-Pentylphenyl-sulfonylchlorid
   [4-(4-Pentylphenyl-sulfonyl)-piperazin-1-yl]-[4-(5-methyl-[1,2,4]oxadiazol-3-yl)-phenyl]-methanon;
mit 4-Butylphenyl-sulfonylchlorid
   [4-(4-Butylphenyl-sulfonyl)-piperazin-1-yl]-[4-(5-methyl-[1,2,4]oxadiazol-3-yl)-phenyl]-methanon;
mit 3-Chlor-4-methylphenyl-sulfonylchlorid
   [4-(3-Chlor-4-methylphenyl-sulfonyl)-piperazin-1-yl]-[4-(5-methyl-[1,2,4]oxadiazol-3-yl)-phenyl]-methanon;

### Beispiel 2

Eine Lösung von 100 mg [4-(6-Chlornaphthalin-2-sulfonyl)-piperazin-1-yl]-[4-(5-methyl-[1,2,4]oxadiazol-3-yl)-phenyl]-methanon in 5 ml Methanol wird mit 100 mg Raney-Nickel und einem Tropfen Essigsäure versetzt und bis zum Stillstand bei Normaldruck und Raumtemperatur hydriert. Nach Entfernen des Katalysators und des Lösungsmittels erhält man 4-[4-(6-Chlornaphthalin-2-sulfonyl)-piperazin-1-carbonyl]-benzamidin, Acetat, FAB 457.

Analog erhält man aus den unter Beispiel 1 aufgeführten Methanonderivaten die nachstehenden Verbindungen
4-[4-(4-Biphenylylsulfonyl)-piperazin-1-carbonyl]-benzamidin, Acetat, FAB 449;
4-[4-(2-Naphthylsulfonyl)-piperazin-1-carbonyl]-benzamidin, Acetat, El 405 (M⁺ - NH₂);
4-[4-(4-Propylphenylsulfonyl)-piperazin-1-carbonyl]-benzamidin, Acetat, FAB 415;
4-[4-(2-Phenylvinylsulfonyl)-piperazin-1-carbonyl]-benzamidin, Acetat, FAB 399;
4-[4-(3-Amino-4-chlorphenylsulfonyl)-piperazin-1-carbonyl]-benzamidin, Acetat, FAB 422;
4-[4-(2-Amino-4-methoxyphenylsulfonyl)-piperazin-1-carbonyl]-benzamidin, Acetat, FAB 418;
4-[4-(4-Tolylsulfonyl)-piperazin-1-carbonyl]-benzamidin, Acetat, FAB 387;
4-[4-(Decylsulfonyl)-piperazin-1-carbonyl]-benzamidin, Acetat, FAB 437;
4-[4-(Benzylsulfonyl)-piperazin-1-carbonyl]-benzamidin, Acetat, FAB 387;
4-[4-(3-Amino-6-methylbenzylsulfonyl)-piperazin-1-carbonyl]-benzamidin, Acetat, FAB 402;
4-[4-(2,3-Dichlorphenyl-sulfonyl)-piperazin-1-carbonyl]-benzamidin, Acetat, FAB 441;
4-[4-(3,4-Dichlorphenyl-sulfonyl)-piperazin-1-carbonyl]-benzamidin, Acetat, FAB 441;
4-[4-(Phenyl-sulfonyl)-piperazin-1-carbonyl]-benzamidin, Acetat, FAB 373;
4-[4-(3-Bromphenyl-sulfonyl)-piperazin-1-carbonyl]-benzamidin, Acetat, FAB 451,453;
4-[4-(3,4-Dimethoxyphenyl-sulfonyl)-piperazin-1-carbonyl]-benzamidin, Acetat, FAB 433;
4-[4-(4-Acetamido-3-chlorphenyl-sulfonyl)-piperazin-1-carbonyl]-benzamidin, Acetat, FAB 464;
4-[4-(4-Chlor-2,5-dimethylphenyl-sulfonyl)-piperazin-1-carbonyl]-benzamidin, Acetat, FAB 435;
4-[4-(3-Tolylsulfonyl)-piperazin-1-carbonyl]-benzamidin, Acetat, FAB 387;
4-[4-(2-Methoxy-5-methylphenyl-sulfonyl)-piperazin-1-carbonyl]-benzamidin, Acetat, FAB 417;
4-[4-(3-Chlorphenyl-sulfonyl)-piperazin-1-carbonyl]-benzamidin, Acetat, FAB 407;
4-[4-(4-Methoxyphenyl-sulfonyl)-piperazin-1-carbonyl]-benzamidin, Acetat, FAB 402;
4-[4-(2-Thienyl-sulfonyl)-piperazin-1-carbonyl]-benzamidin, Acetat, FAB 379;
4-[4-(4-Chlorphenyl-sulfonyl)-piperazin-1-carbonyl]-benzamidin, Acetat, FAB 407;
4-[4-(Isopropylsulfonyl)-piperazin-1-carbonyl]-benzamidin, Acetat, FAB 339;
4-[4-(1,2,3,4-Tetrahydrochinolin-8-yl-sulfonyl)-piperazin-1-carbonyl]-benzamidin, Acetat, FAB 428;
4-[4-(4-Aminophenyl-sulfonyl)-piperazin-1-carbonyl]-benzamidin, Acetat, FAB 388;
4-[4-(3-Chlor-6-methoxyphenyl-sulfonyl)-piperazin-1-carbonyl]-benzamidin, Acetat, FAB 437;
4-[4-(4-Acetamidophenyl-sulfonyl)-piperazin-1-carbonyl]-benzamidin, Acetat, FAB 437;
4-[4-(2,2,5,7,8-Pentamethylchroman-6-yl-sulfonyl)-piperazin-1-carbonyl]-benzamidin, Acetat, FAB 499;
4-[4-(Campher-10-yl-sulfonyl)-piperazin-1-carbonyl]-benzamidin, Acetat, FAB 447;
4-{4-[5-(1-Methyl-5-trifluormethyl-3-pyrazolyl)-2-thienyl-sulfonyl]-piperazin-1-carbonyl}-benzamidin, Acetat, FAB 527;
4-[4-(2,5-Dichlorphenyl-sulfonyl)-piperazin-1-carbonyl]-benzamidin, Acetat, FAB 441;
4-[4-(2,4,6-Trimethylphenyl-sulfonyl)-piperazin-1-carbonyl]-benzamidin, Acetat, FAB 415;
4-[4-(2-Methylsulfonylphenyl-sulfonyl)-piperazin-1-carbonyl]-benzamidin, Acetat, FAB 451;
4-[4-(5-Benzamidomethyl-2-thienyl-sulfonyl)-piperazin-1-carbonyl]-benzamidin, Acetat, FAB 512;
4-[4-(Methylsulfonyl)-piperazin-1-carbonyl]-benzamidin, Acetat, El 292 (M⁺ - NH₂);
4-[4-(1,3-Dimethyl-5-chlor-4-pyrazolyl-sulfonyl)-piperazin-1-carbonyl]-benzamidin, Acetat;
4-[4-(3,5-Dimethyl-4-isoxazolyl-sulfonyl)-piperazin-1-carbonyl]-benzamidin, Acetat;
4-[4-(4-Brom-2-ethylphenyl-sulfonyl)-piperazin-1-carbonyl]-benzamidin, Acetat, El 461, 463 (M⁺ - NH₂);
4-[4-(1-Naphthylsulfonyl)-piperazin-1-carbonyl]-benzamidin, Acetat, El 405 (M⁺ - NH₂);
4-[4-(5-Dimethylamino-1-naphthylsulfonyl)-piperazin-1-carbonyl]-benzamidin, Acetat, El 448 (M⁺ - NH₂);
4-[4-(3,4-Difluorphenyl-sulfonyl)-piperazin-1-carbonyl]-benzamidin, Acetat;
4-[4-(4-tert.-Butylphenyl-sulfonyl)-piperazin-1-carbonyl]-benzamidin, Acetat, FAB 429;
4-[4-(4-Ethylphenyl-sulfonyl)-piperazin-1-carbonyl]-benzamidin, Acetat, FAB 401;
4-[4-(4-(1,1-Dimethylpropyl)-phenyl-sulfonyl)-piperazin-1-carbonyl]-benzamidin, Acetat, FAB 442;
4-[4-(4-lsopropylphenyl-sulfonyl)-piperazin-1-carbonyl]-benzamidin, Acetat, FAB 415;
4-[4-(4-Trifluormethylphenyl-sulfonyl)-piperazin-1-carbonyl]-benzamidin, Acetat, FAB 441;
4-[4-(3-Amino-4-methylphenyl-sulfonyl)-piperazin-1-carbonyl]-benzamidin, Acetat, FAB 402;
4-[4-(4-Pentylphenyl-sulfonyl)-piperazin-1-carbonyl]-benzamidin, Acetat, FAB 443;
4-[4-(4-Butylphenyl-sulfonyl)-piperazin-1-carbonyl]-benzamidin, Acetat, FAB 429;
4-[4-(3-Chlor-4-methylphenyl-sulfonyl)-piperazin-1-carbonyl]-benzamidin, Acetat, FAB 421.

### Beispiel 3

Durch Umsetzung mit äquimolaren Mengen Chlorameisensäuremethylester in Pyridin und katalytischen Mengen Dimethylaminopyridin erhält man
aus 4-[4-(6-Chlornaphthalin-2-sulfonyl)-piperazin-1-carbonyl]-benzamidin nach üblicher Aufarbeitung die Verbindung {Imino-[4-(4-(6-Chlornaphthalin-2-sulfonyl)-piperazin-1-carbonyl)-phenyl]-methyl}-carbaminsäuremethylester.

### Beispiel 4

Analog Beispiel 1 erhält man durch Umsetzung von 3-[4-{5-Methyl-[1,2,4]-oxadiazol-3-yl)-phenyl]-1-piperazin-1-yl-propan-1-on [erhältlich aus 3-[4-(5-Methyl-[1,2,4]oxadiazol-3-yl)-phenyl]-1-(4-tert.-butyl-oxy-carbonyl)-piperazin-1-yl-propan-1-on durch Behandlung mit TFA/CH₂Cl₂] und 6-Chlornaphthalin-2-sulfonylchlorid die Verbindung 1-[4-(6-Chlornaphthalin-2-sulfonyl)-piperazin-1-yl]-3-[4-(5-methyl-[1,2,4]oxadiazol-3-yl)-phenyl]-propan-1-on und nach Hydrierung 4-{3-Oxo-3-[4-(6-chlornaphthalin-2-sulfonyl)-piperazin-1yl]-propyl}-benzamidin.

### Beispiel 5

Analog Beispiel 1 und 2 erhält man durch Umsetzung von [3-(5-Methyl-[1,2,4]oxadiazol-3-yl)-phenyl]-piperazin-1-yl-methanon und 5-Chlornaphthalin-2-sulfonylchlorid und anschließender Hydrierung die Verbindung 3-[4-(6-Chlornaphthalin-2-sulfonyl)-piperazin-1-carbonyl]-benzamidin, Acetat, FAB 457.

Analog erhält man durch Umsetzung von [3-(5-Methyl-[1,2,4]oxadiazol-3-yl)-phenyl]-piperazin-1-yl-methanon mit 4-Propylphenyl-sulfonylchlorid und anschließender Hydrierung die Verbindung 3-[4-(4-Propylphenyl-sulfonyl)-piperazin-1-carbonyl]-benzamidin, Acetat, FAB 415.

### Beispiel 6

Analog Beispiel 1 erhält man durch Umsetzung von 2-[4-(5-Methyl-[1,2,4]oxadiazol-3-yl)-phenyl]-1-piperazin-1-yl-ethan-1-on ("B") und 4-Propylphenyl-sulfonylchlorid die Verbindung 1-[4-(4-Propylphenylsulfonyl)-piperazin-1-yl]-2-[4-(5-methyl-[1,2,4]oxadiazol-3-yl)-phenyl]-ethan-1-on und nach Hydrierung 4-{2-Oxo-2-[4-(4-propylphenylsulfonyl)-piperazin-1yl]-ethyl}-benzamidin, FAB 429.

Analog erhält man durch Umsetzung von "B"
mit Decylsulfonylchlorid
   1-[4-(Decylsulfonyl)-piperazin-1-yl]-2-[4-(5-methyl-[1,2,4]oxadiazol-3-yl)-phenyl]-ethan-1-on;
mit Phenylsulfonylchlorid
   1-[4-(Phenylsulfonyl)-piperazin-1-yl]-2-[4-(5-methyl-[1,2,4]oxadiazol-3-yl)-phenyl]-ethan-1-on;
mit 3,4-Dichlorphenyl-sulfonylchlorid
   1-[4-(3,4-Dichlorphenyl-sulfonyl)-piperazin-1-yl]-2-[4-(5-methyl-[1,2,4]oxadiazol-3-yl)-phenyl]-ethan-1-on;
mit Benzylsulfonylchlorid
   1-[4-(Benzylsulfonyl)-piperazin-1-yl]-2-[4-(5-methyl-[1,2,4]oxadiazol-3-yl)-phenyl]-ethan-1-on;
mit 3,4-Dimethoxyphenyl-sulfonylchlorid
   1-[4-(3,4-Dimethoxyphenyl-sulfonyl)-piperazin-1-yl]-2-[4-(5-methyl-[1,2,4]oxadiazol-3-yl)-phenyl]-ethan-1-on;
mit Isopropylsulfonylchlorid
   1-[4-(Isopropyl-sulfonyl)-piperazin-1-yl]-2-[4-(5-methyl-[1,2,4]oxadiazol-3-yl)-phenyl]-ethan-1-on;
mit Campher-10-yl-sulfonylchlorid
   1-[4-(Campher-10-yl-sulfonyl)-piperazin-1-yl]-2-[4-(5-methyl-[1,2,4]oxadiazol-3-yl)-phenyl]-ethan-1-on;
mit 3-Methoxy-4-methoxycarbonyl-2-thienyl-sulfonylchlorid
   1-[4-(3-Methoxy-4-methoxycarbonyl-2-thienyl-sulfonyl)-piperazin-1-yl]-2-[4-(5-methyl-[1,2,4]oxadiazol-3-yl)-phenyl]-ethan-1-on;
mit 2,4,6-Trimethylphenyl-sulfonylchlorid
   1-[4-(2,4,6-Trimethylphenyl-sulfonyl)-piperazin-1-yl]-2-[4-(5-methyl-[1,2,4]oxadiazol-3-yl)-phenyl]-ethan-1-on;
mit 2-Phenylvinyl-sulfonylchlorid
   1 -[4-(2-Phenylvinyl-sulfonyl)-piperazin-1-yl]-2-[4-(5-methyl-[1,2,4]oxadiazol-3-yl)-phenyl]-ethan-1-on;
mit Methylsulfonylchlorid
   1-[4-(Methylsulfonyl)-piperazin-1-yl]-2-[4-(5-methyl-[1,2,4]oxadiazol-3-yl)-phenyl]-ethan-1-on;
mit [2,1,3]-Benzothiadiazol-4-yl-sulfonylchlorid
   1-[4-([2,1,3]-Benzothiadiazol-4-yl-sulfonyl)-piperazin-1-yl]-2-[4-(5-methyl-[1,2,4]oxadiazol-3-yl)-phenyl]-ethan-1-on;
mit 2,4-Dichlorphenyl-sulfonylchlorid
   1-[4-(2,4-Dichlorphenyl-sulfonyl)-piperazin-1-yl]-2-[4-(5-methyl-[1,2,4]oxadiazol-3-yl)-phenyl]-ethan-1-on;
mit 1-Naphthyl-sulfonylchlorid
   1-[4-(1-Naphthyl-sulfonyl)-piperazin-1-yl]-2-[4-(5-methyl-[1,2,4]oxadiazol-3-yl)-phenyl]-ethan-1-on;
mit 2-Naphthyl-sulfonylchlorid
   1-[4-(2-Naphthyl-sulfonyl)-piperazin-1-yl]-2-[4-(5-methyl-[1,2,4]oxadiazol-3-yl)-phenyl]-ethan-1-on;
mit 5-Dimethylamino-1-naphthyl-sulfonylchlorid
   1 -[4-(5-Dimethylamino-1-naphthyl-sulfonyl)-piperazin-1-yl]-2-[4-(5-methyl-[1,2,4]oxadiazol-3-yl)-phenyl]-ethan-1-on;
mit 4-Methylsulfonylphenyl-sulfonylchlorid
   1-[4-(4-Methylsulfonylphenyl-sulfonyl)-piperazin-1-yl]-2-[4-(5-methyl-[1,2,4]oxadiazol-3-yl)-phenyl]-ethan-1-on.

Durch Hydrierung erhält man daraus nachstehende Amidinderivate
4-{2-Oxo-2-[4-(decylsulfonyl)-piperazin-1yl]-ethyl}-benzamidin, Acetat, FAB 450;
4-{2-Oxo-2-[4-(phenylsulfonyl)-piperazin-1yl]-ethyl}-benzamidin, Acetat, FAB 387;
4-{2-Oxo-2-[4-(3,4-dichlorphenyl-sulfonyl)-piperazin-1yl]-ethyl}benzamidin, Acetat, FAB 454;
4-{2-Oxo-2-[4-(benzylsulfonyl)-piperazin-1yl]-ethyl}-benzamidin, Acetat, FAB 401;
4-{2-Oxo-2-[4-(3,4-dimethoxyphenyl-sulfonyl)-piperazin-1yl]-ethyl}benzamidin, Acetat, FAB 447;
4-{2-Oxo-2-[4-(isopropyl-sulfonyl)-piperazin-1yl]-ethyl}-benzamidin, Acetat, FAB 353;
4-{2-Oxo-2-[4-(campher-10-yl-sulfonyl)-piperazin-1yl]-ethyl}benzamidin, Acetat, FAB 353;
4-{2-Oxo-2-[4-(3-methoxy-4-methoxycarbonyl-2-thienyl-sulfonyl)-piperazin-1yl]-ethyl}-benzamidin, Acetat, FAB 481;
4-{2-Oxo-2-[4-(2,4,6-trimethylphenyl-sulfonyl)-piperazin-1yl)-ethyl}benzamidin, Acetat, FAB 429;
4-{2-Oxo-2-[4-(2-Phenylvinyl-sulfonyl)-piperazin-1yl]-ethyl}benzamidin, Acetat, FAB 413;
4-{2-Oxo-2-[4-(methylsulfonyl)-piperazin-1yl]-ethyl}-benzamidin, Acetat, FAB 325;
4-{2-Oxo-2-[4-(2,3-diaminophenylsulfonyl)-piperazin-1yl]-ethyl}benzamidin, Acetat, FAB 415;
4-{2-Oxo-2-[4-(2,4-dichlorphenylsulfonyl)-piperazin-1yl]-ethyl}benzamidin, Acetat, FAB 455;
4-{2-Oxo-2-[4-(1-naphthyl-sulfonyl)-piperazin-1yl]-ethyl}-benzamidin, Acetat, FAB 437;
4-{2-Oxo-2-[4-(2-naphthyl-sulfonyl)-piperazin-1yl]-ethyl}-benzamidin, Acetat, FAB 437;
4-{2-Oxo-2-[4-(5-dimethylamino-1-naphthyl-sulfonyl)-piperazin-1yl]-ethyl}-benzamidin, Acetat, FAB 480;
4-{2-Oxo-2-[4-(4-methylsulfonylphenyl-sulfonyl)-piperazin-1yl]-ethyl}benzamidin, Acetat, FAB 465.

### Beispiel 7

Analog Beispiel 1 erhält man durch Umsetzung von "A"
mit 4-Biphenylyl-carbonsäurechlorid
   [4-(4-Phenyl-benzoyl)-piperazin-1-yl]-[4-(5-methyl-[1,2,4]oxadiazol-3-yl)-phenyl]-methanon;
mit Cyclopentyl-carbonsäurechlorid
   [4-(Cyclopentylcarbonyl)-piperazin-1-yl]-[4-(5-methyl-[1,2,4]oxadiazol-3-yl)-phenyl]-methanon;
mit Phenoxy-acetylchlorid
   [4-(Phenoxyacetyl)-piperazin-1-yl]-[4-(5-methyl-[1,2,4]oxadiazol-3-yl)-phenyl]-methanon;
mit 1-Naphthyl-carbonsäurechlorid
   [4-(1-Naphthylcarbonyl)-piperazin-1-yl]-[4-(5-methyl-[1,2,4]oxadiazol-3-yl)-phenyl]-methanon;
mit 2-Naphthyl-carbonsäurechlorid
   [4-(2-Naphthylcarbonyl)-piperazin-1-yl]-[4-(5-methyl-[1,2,4]oxadiazol-3-yl)-phenyl]-methanon;
mit Nicotinoylchlorid
   [4-(Nicotinoyl)-piperazin-1-yl]-[4-(5-methyl-[1,2,4]oxadiazol-3-yl)-phenyl]-methanon;
mit 3-Nitro-benzoylchlorid
   [4-(3-Nitro-benzoyl)-piperazin-1-yl]-[4-(5-methyl-[1,2,4]oxadiazol-3-yl)-phenyl]-methanon;
mit Benzo-[b]thiophen-2-carbonsäurechlorid
   [4-(Benzo-[b]thiophen-2-carbonyl)-piperazin-1-yl]-[4-(5-methyl-[1,2,4]oxadiazol-3-yl)-phenyl]-methanon;
mit 4-Trifluormethoxy-benzoylchlorid
   [4-(4-Trifluormethoxy-benzoyl)-piperazin-1-yl]-[4-(5-methyl-[1,2,4]oxadiazol-3-yl)-phenyl]-methanon;
mit 2,5-Dimethoxyphenyl-acetylchlorid
   [4-(2,5-Dimethoxyphenyl-acetyl)-piperazin-1-yl]-[4-(5-methyl-[1,2,4]oxadiazol-3-yl)-phenyl]-methanon;
mit 4-Chlorphenyl-acetylchlorid
   [4-(4-Chlorphenyl-acetyl)-piperazin-1-yl]-[4-(5-methyl-[1,2,4]oxadiazol-3-yl)-phenyl]-methanon;
mit 1,3-Benzodioxol-5-carbonsäurechlorid
   [4-(1,3-Benzodioxol-5-carbonyl)-piperazin-1 -yl]-[4-(5-methyl-[1,2,4]oxadiazol-3-yl)-phenyl]-methanon;
mit 3,4-Dichlorbenzoylchlorid
   [4-(3,4-Dichlorbenzoyl)-piperazin-1-yl]-[4-(5-methyl-[1,2,4]oxadiazol-3-yl)-phenyl]-methanon;
mit Chlorameisensäureisobutylester
   [4-(lsobutyloxycarbonyl)-piperazin-1-yl]-[4-(5-methyl-[1,2,4]oxadiazol-3-yl)-phenyl]-methanon.

Durch Hydrierung erhält man daraus nachstehende Amidinderivate
4-[4-(4-Phenyl-benzoyl)-piperazin-1-carbonyl]-benzamidin, Acetat, FAB 413;
4-[4-(Cyclopentylcarbonyl)-piperazin-1-carbonyl]-benzamidin, Acetat, FAB 329;
4-[4-(Phenoxyacetyl)-piperazin-1-carbonyl]-benzamidin, Acetat, FAB 367;
4-[4-(1-Naphthylcarbonyl)-piperazin-1-carbonyl]-benzamidin, Acetat, FAB 387;
4-[4-(2-Naphthylcarbonyl)-piperazin-1-carbonyl]-benzamidin, Acetat, FAB 387;
4-[4-(Nicotinoyl)-piperazin-1-carbonyl]-benzamidin, Acetat, FAB 338;
4-[4-(3-Aminobenzoyl)-piperazin-1-carbonyl]-benzamidin;
4-[4-(Benzo-[b]thiophen-2-carbonyl)-piperazin-1-carbonyl]-benzamidin, Acetat, FAB 393;
4-[4-(4-Trifluormethoxy-benzoyl)-piperazin-1-carbonyl]-benzamidin, Acetat, FAB 421;
4-[4-(2,5-Dimethoxyphenyl-acetyl)-piperazin-1-carbonyl]-benzamidin, Acetat, FAB 411;
4-[4-(4-Chlorphenyl-acetyl)-piperazin-1-carbonyl]-benzamidin, Acetat, FAB 385;
4-[4-(1,3-Benzodioxol-5-carbonyl)-piperazin-1-carbonyl]-benzamidin, Acetat, FAB 381;
4-[4-(3,4-Dichlorbenzoyl)-piperazin-1-carbonyl]-benzamidin, Acetat, FAB 381;
4-[4-(lsobutyloxycarbonyl)-piperazin-1-carbonyl]-benzamidin, Acetat, FAB 333.

### Beispiel 8

Durch Umsetzung mit äquimolaren Mengen Acetylchlorid in Pyridin und katalytischer Mengen Dimethylaminopyridin erhält man nach üblicher Aufarbeitung
aus 4-[4-(6-Chlornaphthalin-2-sulfonyl)-piperazin-1-carbonyl]-benzamidin
   N-{Imino-4-[4-(6-Chlornaphthalin-2-sulfonyl)-piperazin-1-carbonyl]-phenyl-methyl}-acetamid.

### Beispiel 9

Durch Umsetzung äquimolarer Mengen 4-Cyanbenzylbromid, BOC-Piperazin und Triethylamin in Dichlormethan erhält man 1-(4-Cyan-benzyl)-4-(tert.-butyloxycarbonyl)-piperazin. Durch Umsetzung mit
a) Hydroxylaminhydrochlorid, Triethylamin in Ethanol und
b) Acetanhydrid
erhält man 1-[4-(5-Methyl[1,2,4]oxadiazol-3-yl]-benzyl]-4-(tert.butyloxycarbonyl)-piperazin.

Nach Abspaltung der BOC-Gruppe mit TFA in CH₂Cl₂ erhält man analog Beispiel 1 und 2 durch Umsetzung von 1-[4-(5-Methyl-[1,2,4]oxadiazol-3-yl]-benzyl]-piperazin mit 6-Chlornaphthalin-2-sulfonylchlorid, anschließender Hydrierung und üblicher Aufarbeitung die Verbindung 4-[(6-Chlornaphthalin-2-sulfonyl)-piperazin-1-ylmethyl]-benzamidin.

Analog erhält man die nachstehenden Verbindungen
4-[(4-Biphenylyl-sulfonyl)-piperazin-1-ylmethyl]-benzamidin,
4-[(2-Naphthyl-sulfonyl)-piperazin-1-ylmethyl]-benzamidin,
4-[(4-Propylphenyl-sulfonyl)-piperazin-1-ylmethyl]-benzamidin und
4-[(2-Phenylvinyl-sulfonyl)-piperazin-1-ylmethyl]-benzamidin.

### Beispiel 10

Durch Umsetzung von äquimolaren Mengen 4-(5-Methyl-[1,2,4]oxadiazol-3-yl]-benzoesäure, Phosphorsäurediphenylesterazid und Triethylamin in DMF erhält man nach üblicher Aufarbeitung 4-(5-Methyl-[1,2,4]oxadiazol-3-yl)-benzoylazid.
Durch Erhitzen mit BOC-Piperazin in Toluol erhält man in einer Umlagerungsreaktion nach üblicher Aufarbeitung 1-BOC-4-[4-(5-Methyl-[1,2,4]-oxadiazol-3-yl)-phenylcarbamoyl]-piperazin. Durch Abspaltung der BOC-Gruppe mit TFA in CH₂Cl₂ erhält man 4-[4-(5-Methyl-[1,2,4]oxadiazol-3-yl)-phenylcarbamoyl]-piperazin ("C").

Durch Umsetzung von "C" mit 6-Chlornaphthalin-sulfonylchlorid und anschließender Hydrierung erhält man analog Beispiel 1 und 2 die Verbindung 4-(6-Chlornaphthalin-2-sulfonyl)-piperazin-1-carbonsäure-N-(4-amidino-phenyl)-amid.

Analog erhält man die nachstehenden Verbindungen
4-(4-Biphenylylsulfonyl)-piperazin-1-carbonsäure-N-(4-amidinophenyl)-amid,
4-(2-Naphthyl-sulfonyl)-piperazin-1-carbonsäure-N-(4-amidinophenyl)-amid,
4-(4-Propylphenyl-sulfonyl)-piperazin-1-carbonsäure-N-(4-amidinophenyl)-amid und
4-(2-Phenylvinyl-sulfonyl)-piperazin-1-carbonsäure-N-(4-amidinophenyl)-amid.

### Beispiel 11

Durch Umsetzung äquimolarer Mengen 1-BOC-4-[4-(5-Methyl-[1,2,4]-oxadiazol-3-yl)-phenylcarbamoyl]-piperazin, Bromessigsäuremethylester und Kalium-tert.butylat in DMF erhält man nach üblicher Aufarbeitung die Verbindung {(4-BOC-piperazin-1-carbonyl)-[4-(5-methyl-[1,2,4]oxadiazol-3-yl)-phenyl]-amino}-essigsäuremethylester.
Durch Reaktion mit
a) HCl/Dioxan und b) NaOH erhält man die Verbindung {(Piperazin-1-carbonyl)-[4-(5-methyl-[1,2,4]oxadiazol-3-yl)-phenyl]-amino}-essigsäuremethylester.
Durch Umsetzung mit 6-Chlornaphthalin-sulfonylchlorid erhält man analog Beispiel 1 die Verbindung {[4-(6-Chlornaphthalin-2-sulfonyl)-piperazin-1-carbonyl]-[4-(5-methyl-[1,2,4]oxadiazol-3-yl)-phenyl]-amino}-essigsäuremethylester.
Durch Hydrierung an Raney-Nickel erhält man daraus {[4-(6-Chlornaphthalin-2-sulfonyl)-piperazin-1-carbonyl]-[4-amidinophenyl]-amino}-essigsäuremethylester.
Die Spaltung des Methylesters erfolgt durch Behandlung mit NaOH in Methanol/Wasser. Nach üblicher Aufarbeitung erhält man die {[4-(6-Chlornaphthalin-2-sulfonyl)-piperazin-1-carbonyl]-[4-amidinophenyl]-amino}-essigsäure.

Analog erhält man nachstehender Verbindungen
{[4-(4-Biphenylyl-sulfonyl)-piperazin-1-carbonyl]-[4-amidinophenyl]-amino}-essigsäure,
{[4-(2-Naphthyl-sulfonyl)-piperazin-1-carbonyl]-[4-amidinophenyl]-amino}-essigsäure,
{[4-(4-Propylphenyl-sulfonyl)-piperazin-1-carbonyl]-[4-amidinophenyl]-amino}-essigsäure und
{[4-(2-phenylvinyl-sulfonyl)-piperazin-1-carbonyl]-[4-amidinophenyl]-amino}-essigsäure.

### Beispiel 12

Durch Umsetzung äquimolarer Mengen 4-(5-Methyl-[1,2,4]oxadiazol-3-yl)-phenylessigsäure, Methyliodid und Kaliumcarbonat erhält man 4-(5-Methyl-[1,2,4]oxadiazol-3-yl)-phenylessigsäuremethylester ("D").
Durch Erhitzen äquimolarer Mengen von BOC-Piperazin und Chloracetylchlorid in Toluol erhält man nach üblicher Aufarbeitung 1-BOC-4-Chlormethylcarbonyl-piperazin ("E").
Durch Umsetzung von "D" und "E" mit NaH in DMF erhält man nach üblicher Aufarbeitung die Verbindung 4-(4-BOC-piperazin-1-yl)-2-[4-(5-Methyl-[1,2,4]oxadiazol-3-yl)-phenyl]-4-oxo-buttersäuremethylester.
Durch Reaktion mit
a) HCl/Dioxan und b) NaOH erhält man die Verbindung 4-(Piperazin-1-yl)-2-[4-(5-Methyl-[1,2,4]oxadiazol-3-yl)-phenyl]-4-oxo-buttersäuremethylester. Durch Umsetzung mit 6-Chlornaphthalin-sulfonylchlorid erhält man analog Beispiel 1 die Verbindung 4-[4-(6-Chlornaphthalin-2-sulfonyl)-piperazin-1-yl]-2-[4-(5-Methyl-[1,2,4]oxadiazol-3-yl)-phenyl]-4-oxo-buttersäuremethylester.
Durch Hydrierung analog Beispiel 2 erhält man daraus die Verbindung 4-[4-(6-Chlornaphthalin-2-sulfonyl)-piperazin-1-yl]-2-(4-amidinophenyl)-4-oxo-buttersäuremethylester.

Die Spaltung des Methylesters erfolgt durch Behandlung mit NaOH in Methanol/Wasser. Nach üblicher Aufarbeitung erhält man 4-[4-(6-Chlornaphthalin-2-sulfonyl)-piperazin-1-yl]-2-(4-amidinophenyl)-4-oxobuttersäure.

Analog erhält man nachstehende Verbindungen
4-[4-(4-Biphenylyl-sulfonyl)-piperazin-1-yl]-2-(4-amidinophenyl)-4-oxo-buttersäure,
4-[4-(2-Naphthyl-sulfonyl)-piperazin-1-yl]-2-(4-amidinophenyl)-4-oxo-buttersäure,
4-[4-(4-Propylphenyl-sulfonyl)-piperazin-1-yl]-2-(4-amidinophenyl)-4-oxo-buttersäure und
4-[4-(2-Phenylvinyl-sulfonyl)-piperazin-1-yl]-2-(4-amidinophenyl)-4-oxo-buttersäure.

### Beispiel 13

Durch Umsetzung äquimolarer Mengen "A" und Phenylisocyanat in Dichlormethan bei Raumtemperatur erhält man nach üblicher Aufarbeitung die Verbindung 4-[4-(5-Methyl-[1,2,4]oxadiazol-3-yl)-benzoyl]-piperazin-1-carbonsäure-N-phenyl-amid.

Analog erhält man durch Umsetzung von "A"
mit 4-Trifluormethylphenylisocyanat
   4-[4-(5-Methyl-[1,2,4]oxadiazol-3-yl)-benzoyl]-piperazin-1-carbonsäure-N-(4-trifluormethylphenyl)-amid;
mit Butylisocyanat
   4-[4-(5-Methyl-[1,2,4]oxadiazol-3-yl)-benzoyl]-piperazin-1-carbonsäure-N-butyl-amid;
mit 1-Naphthylisocyanat
   4-[4-(5-Methyl-[1,2,4]oxadiazol-3-yl)-benzoyl]-piperazin-1-carbonsäure-N-(1-naphthyl)-amid;
mit 4-Methoxyphenylisocyanat
   4-[4-(5-Methyl-[1,2,4]oxadiazol-3-yl)-benzoyl]-piperazin-1-carbonsäure-N-(4-methoxyphenyl)-amid;
mit 4-Nitrophenylisocyanat
   4-[4-(5-Methyl-[1,2,4]oxadiazol-3-yl)-benzoyl]-piperazin-1-carbonsäure-N-(4-nitrophenyl)-amid;
mit Cyclohexylisocyanat
   4-[4-(5-Methyl-[1,2,4]oxadiazol-3-yl)-benzoyl]-piperazin-1-carbonsäure-N-cyclohexyl-amid;
mit 3-Ethoxycarbonylphenylisocyanat
   4-[4-(5-Methyl-[1,2,4]oxadiazol-3-yl)-benzoyl]-piperazin-1-carbonsäure-N-(3-ethoxycarbonylphenyl)-amid.

Durch Hydrierung analog Beispiel 2 erhält man daraus die nachstehenden Amidinderivate
4-(4-Amidino-benzoyl)-piperazin-1-carbonsäure-N-phenyl-amid, Acetat, FAB 352;
4-(4-Amidino-benzoyl)-piperazin-1-carbonsäure-N-butyl-amid, Acetat, FAB 332;
4-(4-Amidino-benzoyl)-piperazin-1-carbonsäure-N-(1-naphthyl)-amid, Acetat, FAB 402;
4-(4-Amidino-benzoyl)-piperazin-1-carbonsäure-N-(4-methoxyphenyl)-amid, Acetat, FAB 382;
4-(4-Amidino-benzoyl)-piperazin-1-carbonsäure-N-(4-aminophenyl)-amid, Acetat, FAB 367;
4-(4-Amidino-benzoyl)-piperazin-1-carbonsäure-N-cyclohexyl-amid, Acetat, FAB 358;
4-(4-Amidino-benzoyl)-piperazin-1-carbonsäure-N-(3-ethoxycarbonylphenyl)-amid, Acetat, FAB 424.

### Beispiel 14

Analog Beispiel 1 und 2 erhält man die nachstehenden Verbindungen
3-[4-(2-Naphthylsulfonyl)-piperazin-1-carbonyl]-benzamidin, Acetat, FAB 423;
3-[4-(3-chlor-4-methylphenyl-sulfonyl)-piperazin-1-carbonyl]-benzamidin, Acetat, FAB 421;
3-[4-(2,4,6-trichlorphenyl-sulfonyl)-piperazin-1-carbonyl]-benzamidin, Acetat, FAB 475,477;
3-[4-(3-amino-4-chlorphenyl-sulfonyl)-piperazin-1-carbonyl]-benzamidin, Acetat, FAB 422;
3-[4-(4-chlor-phenyl-sulfonyl)-piperazin-1-carbonyl]-benzamidin, Acetat, FAB 407;
3-[4-(3-trifluormethyl-phenyl-sulfonyl)-piperazin-1-carbonyl]-benzamidin, Acetat, FAB 441;
3-[4-(4-biphenylyl-sulfonyl)-piperazin-1-carbonyl]-benzamidin, Acetat, FAB 449;
4-[4-(3,5-Dimethoxyphenyl-sulfonyl)-piperazin-1-carbonyl]-benzamidin, Acetat, FAB 433;
4-[4-(Dibenzofuran-2-yl-sulfonyl)-piperazin-1-carbonyl]-benzamidin, Acetat, FAB 463;
4-[4-(3-Fluor-4-methoxyphenyl-sulfonyl)-piperazin-1-carbonyl]-benzamidin, Acetat, FAB 421;
4-[4-(2,4-Dichlor-6-methoxyphenyl-sulfonyl)-piperazin-1-carbonyl]-benzamidin, Acetat, FAB 471;
4-(4-Benzylpiperazin-1-carbonyl)-benzamidin, Acetat, FAB 323;
4-[4-(2-Naphthylmethyl)-piperazin-1-carbonyl]-benzamidin, Acetat, FAB 373;
4-[4-(4-Methoxyphenylmethyl)-piperazin-1-carbonyl]-benzamidin, Diacetat, FAB 353;
4-[4-(4-Methoxycarbonylphenyl-sulfonyl)-piperazin-1-carbonyl]-benzamidin, Acetat, FAB 431;
4-[4-(4-Propylphenyl-sulfonyl)-piperazin-1-carbonyl]-3-methylbenzamidin, Acetat, FAB 429;
4-[4-(2-Naphthyl-sulfonyl)-piperazin-1-carbonyl]-3-methyl-benzamidin, Acetat, FAB 437;
4-[4-(6-Chlor-2-naphthyl-sulfonyl)-piperazin-1-carbonyl]-3-methyl-benzamidin, Acetat, FAB 471;
4-[4-(7-Methoxy-2-naphthyl-sulfonyl)-piperazin-1-carbonyl]-benzamidin, Acetat, FAB 453;
4-[4-(3,5-Dimethoxyphenylmethyl)-piperazin-1-carbonyl]-benzamidin, Acetat, FAB 383;

### Beispiel 15

Analog Beispiel 6 erhält man die nachstehenden Verbindungen
4-{3-Oxo-3-[4-(butylsulfonyl)-piperazin-1-yl]-propyl}-benzamidin, Acetat, FAB 381;
4-{3-Oxo-3-[4-(4-propylphenyl-sulfonyl)-piperazin-1-yl]-propyl}-benzamidin, Acetat, FAB 443;
4-{3-Oxo-3-[4-(6-chlor-2-naphthyl-sulfonyl)-piperazin-1-yl]-propyl}-benzamidin, Acetat, FAB 485;
4-{3-Oxo-3-[4-(2-naphthyl-sulfonyl)-piperazin-1-yl]-propyl}-benzamidin, Acetat, FAB 451;
4-{3-Oxo-3-[4-(3-chlor-4-methylphenyl-sulfonyl)-piperazin-1-yl]-propyl}-benzamidin, Acetat, FAB 449;
4-{3-Oxo-3-[4-(4-chlor-phenyl-sulfonyl)-piperazin-1-yl]-propyl}-benzamidin, Acetat, FAB 435;
4-{3-Oxo-3-[4-(4-biphenylyl-sulfonyl)-piperazin-1-yl]-propyl}-benzamidin, Acetat, FAB 477;
4-{3-Oxo-3-[4-(2,4,6-trimethyl-phenyl-sulfonyl)-piperazin-1-yl]-propyl}-benzamidin, Acetat, FAB 443;
3-{3-Oxo-3-[4-(butylsulfonyl)-piperazin-1-yl]-propyl}-benzamidin, Acetat, FAB 381;
3-{3-Oxo-3-[4-(4-methoxyphenyl-sulfonyl)-piperazin-1-yl]-propyl}-benzamidin, Acetat, FAB 431;
3-{3-Oxo-3-[4-(4-chlorphenyl-sulfonyl)-piperazin-1-yl]-propyl}-benzamidin, Acetat, FAB 435;
3-{3-Oxo-3-[4-(4-isopropylphenyl-sulfonyl)-piperazin-1-yl]-propyl}-benzamidin, Acetat, FAB 443;
3-{3-Oxo-3-[4-(2,4,6-trimethylphenyl-sulfonyl)-piperazin-1-yl]-propyl}-benzamidin, Acetat, FAB 443;
3-{3-Oxo-3-[4-(3-chlor-4-methylphenyl-sulfonyl)-piperazin-1-yl]-propyl}-benzamidin, Acetat, FAB 449;
3-{3-Oxo-3-[4-(6-chlor-2-naphthyl-sulfonyl)-piperazin-1-yl]-propyl}-benzamidin, Acetat, FAB 485;
3-{3-Oxo-3-[4-(2-naphthyl-sulfonyl)-piperazin-1-yl]-propyl}-benzamidin, Acetat, FAB 451;
3-{3-Oxo-3-[4-(4-biphenylyl-sulfonyl)-piperazin-1-yl]-propyl}-benzamidin, Acetat, FAB 477;

### Beispiel 16

Analog Beispiel 13 erhält man durch Umsetzung von 4-(5-Methyl-[1,2,4]-oxadiazol-3-yl)-phenylisocyanat ("F")
mit 1-(2-Naphthyl-sulfonyl)-piperazin
   4-(2-Naphthyl-sulfonyl)-piperazin-1-carbonsäure-N-[4-(5-methyl-[1,2,4]-oxadiazol-3-yl)-phenyl]-amid 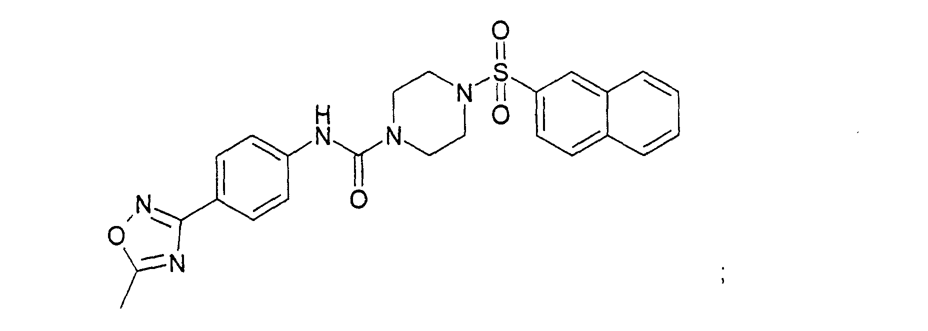
mit 1-(2-Phenylvinyl-sulfonyl)-piperazin
   4-(2-Phenylvinyl-sulfonyl)-piperazin-1-carbonsäure-N-[4-(5-methyl-[1,2,4]-oxadiazol-3-yl)-phenyl]-amid;
mit 1-(4-Propylphenyl-sulfonyl)-piperazin
   4-(4-Propylphenyl-sulfonyl)-piperazin-1-carbonsäure-N-[4-(5-methyl-[1,2,4]-oxadiazol-3-yl)-phenyl]-amid;
mit 1-(4-Chlorphenyl-sulfonyl)-piperazin
   4-(4-Chlorphenyl-sulfonyl)-piperazin-1-carbonsäure-N-[4-(5-methyl-[1,2,4]-oxadiazol-3-yl)-phenyl]-amid;
mit 1-(2,4,6-Trimethylphenyl-sulfonyl)-piperazin
   4-(2,4,6-Trimethylphenyl-sulfonyl)-piperazin-1-carbonsäure-N-[4-(5-methyl-[1,2,4]-oxadiazol-3-yl)-phenyl]-amid;
mit 1-(6-Chlor-2-naphthyl-sulfonyl)-piperazin
   4-(6-Chlor-2-naphthyl-sulfonyl)-piperazin-1-carbonsäure-N-[4-(5-methyl-[1,2,4]-oxadiazol-3-yl)-phenyl]-amid.

Durch Hydrierung analog Beispiel 2 erhält man daraus die nachstehenden Amidinderivate
4-(2-Naphthyl-sulfonyl)-piperazin-1-carbonsäure-N-(4-amidinophenyl)-amid, Acetat, FAB 438;
4-(2-Phenylvinyl-sulfonyl)-piperazin-1-carbonsäure-N-(4-amidinophenyl)-amid, Acetat, FAB 414;
4-(4-Propylphenyl-sulfonyl)-piperazin-1-carbonsäure-N-(4-amidinophenyl)-amid, Acetat, FAB 430;
4-(4-Chlorphenyl-sulfonyl)-piperazin-1-carbonsäure-N-(4-amidinophenyl)-amid, Acetat, FAB 422;
4-(2,4,6-Trimethylphenyl-sulfonyl)-piperazin-1-carbonsäure-N-(4-amidino-phenyl)-amid, Acetat, FAB 430;
4-(6-Chlor-2-naphthyl-sulfonyl)-piperazin-1-carbonsäure-N-(4-amidino-phenyl)-amid, Acetat, FAB 472.

### Beispiel 17

Analog Beispiel 1 erhält man durch Umsetzung von 1-[4-(5-Methyl-[1,2,4]oxadiazol-3-yl)-benzyl]-piperazin ("G")
mit 4-Propylphenyl-sulfonylchlorid
   1-[4-(5-Methyl-[1,2,4]oxadiazol-3-yl)-benzyl]-4-(4-propylphenylsulfonyl)-piperazin;
mit 4-Methoxyphenyl-sulfonylchlorid
   1-[4-(5-Methyl-[1,2,4]oxadiazol-3-yl)-benzyl]-4-(4-methoxyphenylsulfonyl)-piperazin;
mit 4-Biphenylyl-sulfonylchlorid
   1-[4-(5-Methyl-[1,2,4]oxadiazol-3-yl)-benzyl]-4-(4-biphenylyl-sulfonyl)-piperazin;
mit 2-Naphthyl-sulfonylchlorid
   1-[4-(5-Methyl-[1,2,4]oxadiazol-3-yl)-benzyl]-4-(2-naphthyl-sulfonyl)-piperazin;
mit 6-Chlor-2-naphthyl-sulfonylchlorid
   1-[4-(5-Methyl-[1,2,4]oxadiazol-3-yl)-benzyl]-4-(6-chlor-2-naphthylsulfonyl)-piperazin;
mit 7-Methoxy-2-naphthyl-sulfonylchlorid
   1-[4-(5-Methyl-[1,2,4]oxadiazol-3-yl)-benzyl]-4-(7-methoxy-2-naphthyl-sulfonyl)-piperazin;
mit 3,5-Dimethoxybenzylchlorid
   1-[4-(5-Methyl-[1,2,4]oxadiazol-3-yl)-benzyl]-4-(3,5-dimethoxybenzyl)-piperazin;
mit 4-Isopropylphenyl-sulfonylchlorid
   1-[4-(5-Methyl-[1,2,4]oxadiazol-3-yl)-benzyl]-4-(4-isopropylphenylsulfonyl)-piperazin;
mit 4-Biphenylyl-carbonsäurechlorid
   1-[4-(5-Methyl-[1,2,4]oxadiazol-3-yl)-benzyl]-4-(4-biphenylyl-carbonyl)-piperazin;
mit 2-Naphthyl-carbonsäurechlorid
   1-[4-(5-Methyl-[1,2,4]oxadiazol-3-yl)-benzyl]-4-(2-naphthyl-carbonyl)-piperazin;
mit 2-Naphthylmethylchlorid
   1-[4-(5-Methyl-[1,2,4]oxadiazol-3-yl)-benzyl]-4-(2-naphthylmethyl)-piperazin.

Durch Hydrierung analog Beispiel 2 erhält man daraus die nachstehenden Amidinderivate
1-(4-Amidinobenzyl)-4-(4-propylphenyl-sulfonyl)-piperazin, Acetat, FAB 401;
1-(4-Amidinobenzyl)-4-(4-methoxyphenyl-sulfonyl)-piperazin, Acetat, FAB 389;
1-(4-Amidinobenzyl)-4-(4-biphenylyl-sulfonyl)-piperazin, Acetat, FAB 435;
1-(4-Amidinobenzyl)-4-(2-naphthyl-sulfonyl)-piperazin, Acetat, FAB 409;
1-(4-Amidinobenzyl)-4-(6-chlor-2-naphthyl-sulfonyl)-piperazin, Acetat, FAB 443;
1-(4-Amidinobenzyl)-4-(7-methoxy-2-naphthyl-sulfonyl)-piperazin, Acetat, FAB 439;
1-(4-Amidinobenzyl)-4-(3,5-dimethoxybenzyl)-piperazin, Acetat, FAB 369;
1-(4-Amidinobenzyl)-4-(4-isopropylphenyl-sulfonyl)-piperazin, Acetat, FAB 441;
1-(4-Amidinobenzyl)-4-(4-biphenylyl-carbonyl)-piperazin, Diacetat, FAB 399;
1-(4-Amidinobenzyl)-4-(2-naphthyl-carbonyl)-piperazin, Diacetat, FAB 373;
1-(4-Amidinobenzyl)-4-(2-naphthylmethyl)-piperazin, Diacetat, FAB 359.

### Beispiel 18

Durch Umsetzung von 4-[4-(6-Chlor-2-Naphthyl-sulfonyl)-piperazin-1-carbonyl]-3-methyl-benzamidin mit Chlorameisensäuremethylester in Dichlormethan erhält man nach üblicher Aufarbeitung die Verbindung (Imino-{4-[4-(6-Chlor-2-naphthyl-sulfonyl)-piperazin-1-carbonyl]-phenyl}methyl)-carbaminsäuremethylester 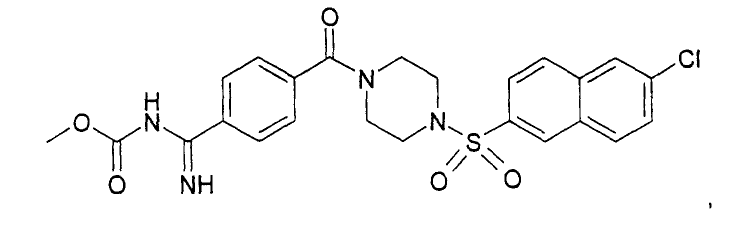 FAB 515.

### Beispiel 19

Analog Beispiel 1 erhält man durch Umsetzung von 1-[4-(5-Methyl-[1,2,4]oxadiazol-3-yl)-phenyl]-piperazin ("H")
mit 4-Propylphenyl-sulfonylchlorid
   1-[4-(5-Methyl-[1,2,4]oxadiazol-3-yl)-phenyl]-4-(4-propylphenyl-sulfonyl)-piperazin;
mit 4-Butyl-sulfonylchlorid
   1-[4-(5-Methyl-[1,2,4]oxadiazol-3-yl)-phenyl]-4-(4-butyl-sulfonyl)-piperazin;
mit 4-Methoxyphenyl-sulfonylchlorid
   1-[4-(5-Methyl-[1,2,4]oxadiazol-3-yl)-phenyl]-4-(4-methoxyphenyl-sulfonyl)-piperazin;
mit 4-Chlorphenyl-sulfonylchlorid
   1-[4-(5-Methyl-[1,2,4]oxadiazol-3-yl)-phenyl]-4-(4-chlorphenyl-sulfonyl)-piperazin;
mit 4-lsopropylphenyl-sulfonylchlorid
   1-[4-(5-Methyl-[1,2,4]oxadiazol-3-yl)-phenyl]-4-(4-isopropylphenyl-sulfonyl)-piperazin;
mit 4-Biphenylyl-sulfonylchlorid
   1-[4-(5-Methyl-[1,2,4]oxadiazol-3-yl)-phenyl]-4-(4-biphenylylphenyl-sulfonyl)-piperazin;
mit 2,4,6-Trimethylphenyl-sulfonylchlorid
   1-[4-(5-Methyl-[1,2,4]oxadiazol-3-yl)-phenyl]-4-(2,4,6-trimethylphenyl-sulfonyl)-piperazin;
mit 3-Chlor-4-methylphenyl-sulfonylchlorid
   1-[4-(5-Methyl-[1,2,4]oxadiazol-3-yl)-phenyl]-4-(3-chlor-4-methylphenyl-sulfonyl)-piperazin;
mit 2-Naphthyl-sulfonylchlorid
   1-[4-(5-Methyl-[1,2,4]oxadiazol-3-yl)-phenyl]-4-(2-naphthyl-sulfonyl)-piperazin;
mit 6-Chlor-2-naphthyl-sulfonylchlorid
   1-[4-(5-Methyl-[1,2,4]oxadiazol-3-yl)-phenyl]-4-(6-chlor-2-naphthyl-sulfonyl)-piperazin.

Durch Hydrierung analog Beispiel 2 erhält man daraus die nachstehenden Amidinderivate
1-(4-Amidinophenyl)-4-(4-propylphenyl-sulfonyl)-piperazin, Acetat, FAB 387;
1-(4-Amidinophenyl)-4-(4-butyl-sulfonyl)-piperazin, Acetat, FAB 325;
1-(4-Amidinophenyl)-4-(4-methoxyphenyl-sulfonyl)-piperazin, Acetat, FAB 375;
1-(4-Amidinophenyl)-4-(4-chlorphenyl-sulfonyl)-piperazin, Acetat, FAB 379;
1-(4-Amidinophenyl)-4-(4-isopropylphenyl-sulfonyl)-piperazin, Acetat, FAB 387;
1-(4-Amidinophenyl)-4-(4-biphenylylphenyl-sulfonyl)-piperazin, Acetat FAB 421;
1-(4-Amidinophenyl)-4-(2,4,6-trimethylphenyl-sulfonyl)-piperazin, Acetat, FAB 387;
1-(4-Amidinophenyl)-4-(3-chlor-4-methylphenyl-sulfonyl)-piperazin, Acetat, FAB 393;
1-(4-Amidinophenyl)-4-(2-naphthyl-sulfonyl)-piperazin, Acetat, FAB 395;
1-(4-Amidinophenyl)-4-(6-chlor-2-naphthyl-sulfonyl)-piperazin, Acetat, FAB 429.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 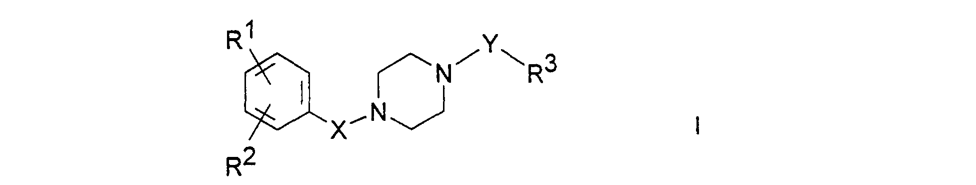 worin
R¹ -C(=NH)-NH₂, das auch einfach durch -COA, -CO-[C(R⁶)₂]ₙ-Ar, -COOA, -OH oder durch eine konventionelle Aminoschutzgruppe substituiert sein kann, 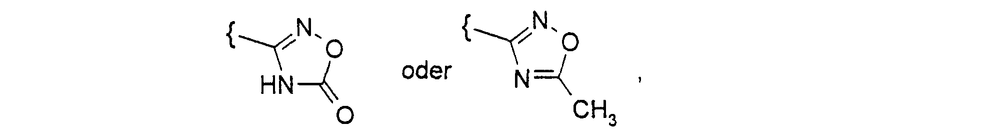
R² H, A, OR⁶, N(R⁶)₂, NO₂, CN, Hal, NHCOA, NHCOAr, NHSO₂A, NHSO₂Ar, COOR⁶, CON(R⁶)₂, CONHAr, COR⁶, COAr, S(O)ₙA oder S(O)ₙAr,
R³ A, Cycloalkyl, -[C(R⁶)₂]ₙAr, -[C(R⁶)₂]ₙ-O-Ar, -[C(R⁶)₂]ₙHet oder -C(R⁶)₂=C(R⁶)₂-Ar,
R⁶ H, A oder Benzyl,
X fehlt, -CO-, -C(R⁶)₂-, -C(R⁶)₂-C(R⁶)₂-, -C(R⁶)₂-CO-, -C(R⁶)₂-C(R⁶)₂-CO-, -C(R⁶)=C(R⁶)-CO-, NR⁶CO-, -N{[C(R⁶)₂]ₙ-COOR⁶}-CO- oder -C(COOR⁶)R⁶-C(R⁶)₂-CO-,
Y -C(R⁶)₂-, -SO₂-, -CO-, -COO- oder -CONR⁶-,
A Alkyl mit 1-20 C-Atomen, worin eine oder zwei CH₂- Gruppen durch O- oder S-Atome oder durch -CR⁶=CR⁶- Gruppen und/oder 1-7 H-Atome durch F ersetzt sein können,
Ar unsubstituiertes oder ein-, zwei- oder dreifach durch A, Ar', OR⁶, N(R⁶)₂, NO₂, CN, Hal, NHCOA, NHCOAr', NHSO₂A, NHSO₂Ar', COOR⁶, CON(R⁶)₂, CONHAr', COR⁶, COAr', S(O)ₙA oder S(O)ₙAr substituiertes Phenyl oder Naphthyl,
Ar' unsubstituiertes oder ein-, zwei- oder dreifach durch A, OR⁶, N(R⁶)₂, NO₂, CN, Hal, NHCOA, COOR⁶, CON(R⁶)₂, COR⁶, oder S(O)ₙA substituiertes Phenyl oder Naphthyl,
Het ein- oder zweikerniges unsubstituiertes oder ein- oder mehrfach durch Hal, A, Ar', COOR⁶, CN, N(R⁶)₂, NO₂, Ar-CONH-CH₂ und/oder Carbonylsauerstoff substituiertes gesättigtes oder ungesättigtes heterocyclisches Ringsystem, welches eines, zwei, drei oder vier gleiche oder verschiedene Heteroatome wie Stickstoff, Sauerstoff und Schwefel enthält,
Hal F, Cl, Br oder I,
n 0, 1 oder 2 bedeutet,
sowie deren Salze.

2. Verbindungen gemäß Anspruch 1
a) 4-[4-(4-Propylphenylsulfonyl)-1-piperazinylcarbonyl]-benzamidin;
b) 4-[4-(3-Amino-4-chlorphenylsulfonyl)-1-piperazinylcarbonyl]-benzamidin;
c) 4-[4-(6-Chlornaphthalin-2-sulfonyl)-1-piperazinylcarbonyl]-benzamidin;
d) 4-[4-(2-Phenylvinylsulfonyl)-1-piperazinylcarbonyl]-benzamidin;
sowie deren Salze.

3. Verfahren zur Herstellung von Verbindungen der Formel I nach Anspruch 1 sowie ihrer Salze, **dadurch gekennzeichnet, daß** man
a) sie aus einem ihrer funktionellen Derivate durch Behandeln mit einem solvolysierenden oder hydrogenolysierenden Mittel in Freiheit setzt, indem man
i) eine Amidinogruppe aus ihrem Oxadiazolderivat durch Hydrogenolyse freisetzt,
ii) eine konventionelle Aminoschutzgruppe durch Behandeln mit einem solvolysierenden oder hydrogenolysierenden Mittel durch Wasserstoff ersetzt oder
eine durch eine konventionelle Schutzgruppe geschützte Aminogruppe in Freiheit setzt,
oder
b) daß man zur Herstellung von Verbindungen der Formel I,
worin R¹ 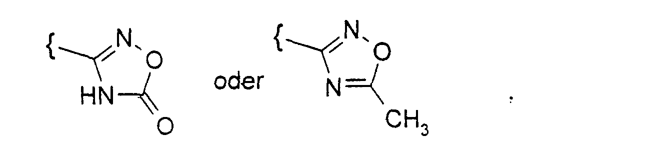
X -CO- oder -C(R⁶)₂-CO-,
und R², R³ und Y die in Anspruch 1 angegebenen Bedeutungen haben,
eine Verbindung der Formel II 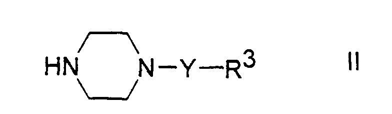 worin
R³ und Y die in Anspruch 1 angegebenen Bedeutungen haben,
mit einer Verbindung der Formel III 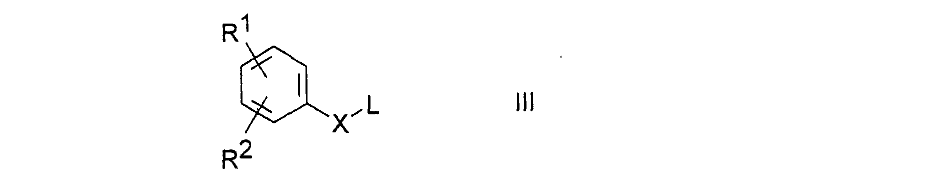 worin R¹ 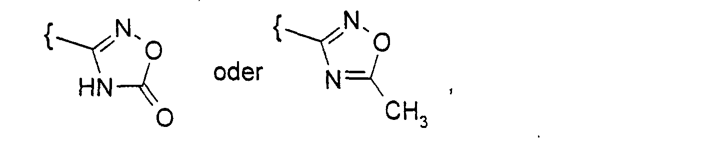
X -CO- oder -C(R⁶)₂-CO- bedeutet,
R² die in Anspruch 1 angegebene Bedeutung hat,
und L Cl, Br, I oder eine freie oder reaktionsfähig funktionell abgewandelte OH-Gruppe bedeutet,
umsetzt,
oder
c) daß man zur Herstellung von Verbindungen der Formel I,
worin R¹ 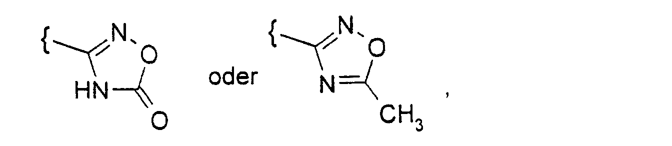
Y -SO₂-, -CO-, -COO- oder -C(R⁶)₂- bedeutet,
und R² und X die in Anspruch 1 angegebenen Bedeutungen haben,
eine Verbindung der Formel IV
L-Y-R³ IV
worin
Y -SO₂-, -CO-, -COO- oder -C(R⁶)₂- bedeutet,
R³ die in Anspruch 1 angegebene Bedeutung hat,
und L Cl, Br, I oder eine freie oder reaktionsfähig funktionell abgewandelte OH-Gruppe bedeutet,
mit einer Verbindung der Formel V 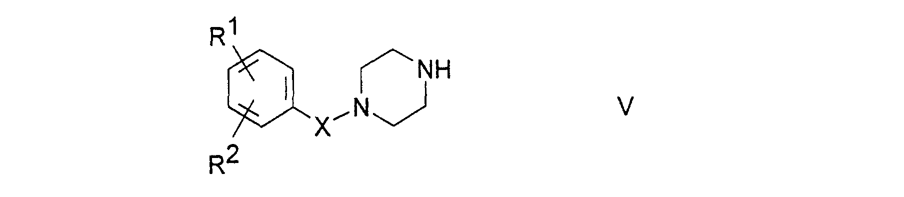 worin R¹ 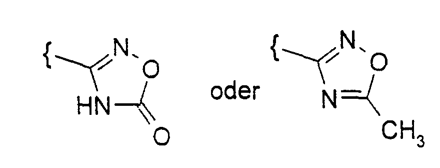 bedeutet,
und R² und X die in Anspruch 1 angegebenen Bedeutungen haben,
umsetzt,
oder
d) daß man zur Herstellung von Verbindungen der Formel I,
worin R¹ 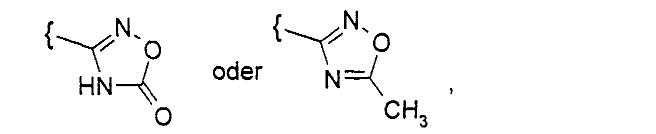
Y -CONH- bedeutet,
und R² und X die in Anspruch 1 angegebenen Bedeutungen haben,
eine Verbindung der Formel VI
R³-N=C=O VI
worin R³ die in Anspruch 1 angegebene Bedeutung hat,
mit einer Verbindung der Formel V 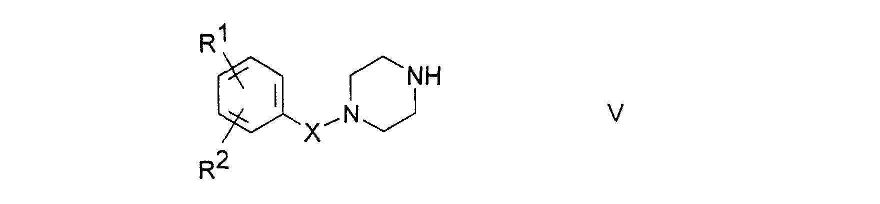 worin R¹ 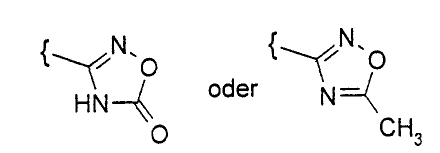 bedeutet,
und R² und X die in Anspruch 1 angegebenen Bedeutungen haben,
umsetzt,
oder
e) daß man zur Herstellung von Verbindungen der Formel I,
worin R¹ -C(=NH)-NH₂ bedeutet,
eine Cyangruppe in eine Amidinogruppe umwandelt,
f) und/oder daß man in einer Verbindung der Formel I einen oder mehrere Rest(e) R¹, R² und/oder R³ in einen oder mehrere Rest(e) R¹, R² und/oder R³ umwandelt,
indem man beispielsweise
i) eine Estergruppe zu einer Carboxygruppe hydrolysiert,
ii) eine Nitrogruppe reduziert,
iii) eine Aminogruppe acyliert,
g) und/oder eine Base oder Säure der Formel I in eines ihrer Salze umwandelt.

4. Verfahren zur Herstellung pharmazeutischer Zubereitungen, **dadurch gekennzeichnet, daß** man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

5. Pharmazeutische Zubereitung, **gekennzeichnet durch** einen Gehalt an mindestens einer Verbindung der Formel I nach Anspruch 1 und/oder einem ihrer physiologisch unbedenklichen Salze.

6. Verbindungen der Formel I nach Anspruch 1 und ihre physiologisch unbedenklichen Salze zur Bekämpfung von Thrombosen, myocardialem Infarkt, Arteriosklerose, Entzündungen, Apoplexie, Angina pectoris, Restenose nach Angioplastie und Claudicatio intermittens.

7. Arzneimittel der Formel I nach Anspruch 1 und ihre physiologisch unbedenklichen Salze als Inhibitoren des Koagulationsfaktors Xa.

8. Verwendung von Verbindungen der Formel I nach Anspruch 1 und/oder ihre physiologisch unbedenklichen Salze zur Herstellung eines Arzneimittels.

9. Verwendung von Verbindungen der Formel I nach Anspruch 1 und/oder ihrer physiologisch unbedenklichen Salze zur Herstellung eines Arzneimittels für die Bekämpfung von Thrombosen, myocardialem Infarkt, Arteriosklerose, Entzündungen, Apoplexie, Angina pectoris, Restenose nach Angioplastie und Claudicatio intermittens.

## Claims

1. Compounds of the formula I 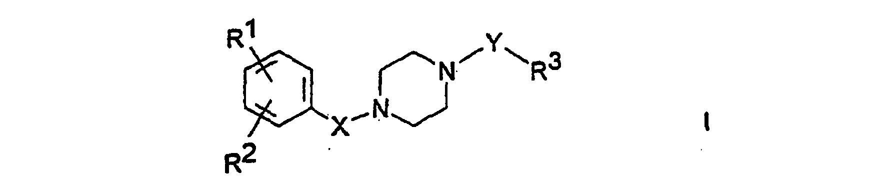 in which
R¹ is -C(=NH)-NH₂, which may also be monosubstituted by -COA, -CO-[C(R⁶)₂]ₙ-Ar, -COOA, -OH or by a conventional amino-protecting group, or 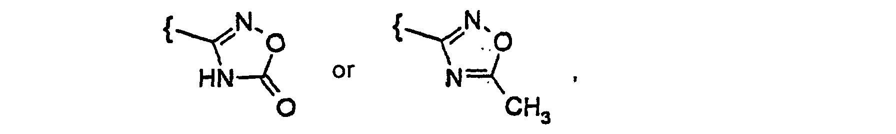
R² is H, A, OR⁶, N(R⁶)₂, NO₂, CN, Hal, NHCOA, NHCOAr, NHSO₂A, NHSO₂Ar, COOR⁶, CON(R⁶)₂, CONHAr, COR⁶, COAr, S(O)ₙA or S(O)ₙAr,
R³ is A, cycloalkyl, -[C(R⁶)₂]Ar, -[C(R⁶)₂]ₙ-O-Ar, -[C(R⁶)₂]ₙHet or -C(R⁶)₂=C(R⁶)₂-Ar,
R⁶ is H, A or benzyl,
X is absent or is -CO-, -C(R⁶)₂-, -C(R⁶)₂-C(R⁶)₂-, -C(R⁶)₂-CO-, -C(R⁶)₂-C(R⁶)₂-CO-, -C(R⁶)=C(R⁶)-CO-, NR⁶CO-, -N{[C(R⁶)₂]ₙ-COOR⁶}-CO- or -C(COOR⁶)R⁶-C(R⁶)₂-CO-,
Y is -C(R⁶)₂-, -SO₂-, -CO-, -COO- or -CONR⁶-,
A is alkyl having 1-20 carbon atoms, in which one or two CH₂ groups may be replaced by O or S atoms or by -CR⁶=CR⁶- groups and/or 1-7 H atoms may be replaced by F,
Ar is phenyl or naphthyl, each of which is unsubstituted or monosubstituted, disubstituted or trisubstituted by A, Ar', OR⁶, N(R⁶)₂, NO₂, CN, Hal, NHCOA, NHCOAr', NHSO₂A, NHSO₂Ar', COOR⁶, CON(R⁶)₂, CONHAr', COR⁶, COAr', S(O)ₙA or S(O)ₙAr,
Ar' is phenyl or naphthyl, each of which is unsubstituted or monosubstituted, disubstituted or trisubstituted by A, OR⁶, N(R⁶)₂, NO₂, CN, Hal, NHCOA, COOR⁶, CON(R⁶)₂, COR⁶ or S(O)ₙA,
Het is a monocyclic or bicyclic, saturated or unsaturated heterocyclic ring system which has one, two, three or four identical or different heteroatoms, such as nitrogen, oxygen and sulfur, and which is unsubstituted or monosubstituted or polysubstituted by Hal, A, Ar', COOR⁶, CN, N(R⁶)₂, NO₂, Ar-CONH-CH₂ and/or carbonyl oxygen,
Hal is F, Cl, Br or I,
n is 0, 1 or 2,
and salts thereof.

2. Compounds according to Claim 1,
a) 4-[4-(4-propylphenylsulfonyl)-1-piperazinylcarbonyl]benzamidine;
b) 4-[4-(3-amino-4-chlorophenylsulfonyl)-1-piperazinylcarbonyl]-benzamidine;
c) 4-[4-(6-chloronaphthalene-2-sulfonyl)-1-piperazinylcarbonyl]-benzamidine;
d) 4-[4-(2-phenylvinylsulfonyl)-1-piperazinylcarbonyl]benzamidine;
and salts thereof.

3. Process for the preparation of compounds of the formula I according to Claim 1 and salts thereof, **characterised in that**
a) they are liberated from one of their functional derivatives by treatment with a solvolysing or hydrogenolysing agent by
i) liberating an amidino group from its oxadiazole derivative by hydrogenolysis,
ii) replacing a conventional amino-protecting group with hydrogen by treatment with a solvolysing or hydrogenolysing agent or
liberating an amino group protected by a conventional protecting group,
or
b) for the preparation of compounds of the formula I
in which R¹ is 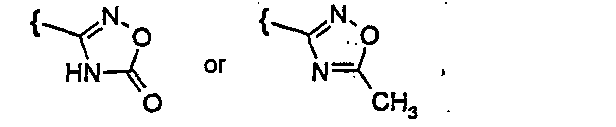
X is -CO- or -C(R⁶)₂-CO-,
and R², R³ and Y are as defined in Claim 1,
a compound of the formula II 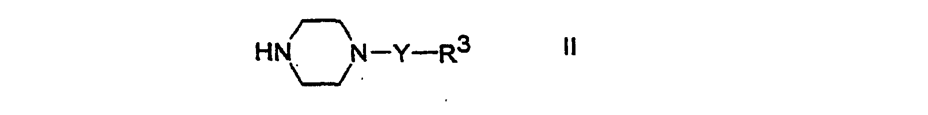 in which
R³ and Y are as defined in Claim 1,
is reacted with a compound of the formula III 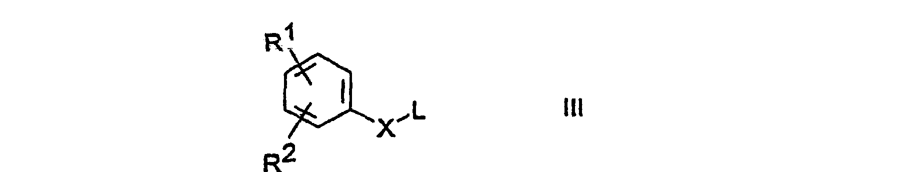 in which R¹ is 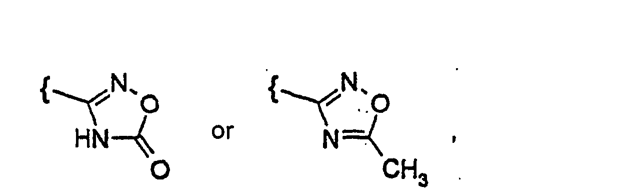
X is -CO- or -C(R⁶)₂-CO-,
R² is as defined in Claim 1,
and L is Cl, Br, I or a free or reactively functionally modified OH group,
or
c) for the preparation of compounds of the formula I
in which R¹ is 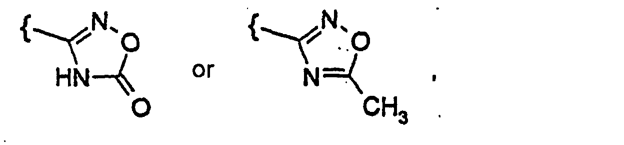
Y is -SO₂-, -CO-, -COO- or -C(R⁶)₂-,
and R² and X are as defined in Claim 1,
a compound of the formula IV
L-Y-R³ IV
in which
Y is -SO₂-, -CO-, -COO- or -C(R⁶)₂-,
R³ is as defined in Claim 1,
and L is Cl, Br, I or a free or reactively functionally modified OH group,
is reacted with a compound of the formula V 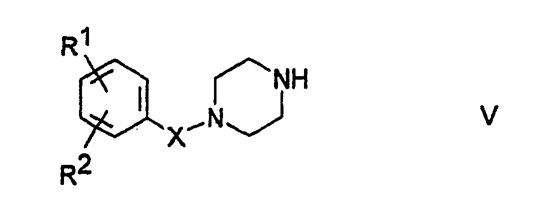 in which R¹ is 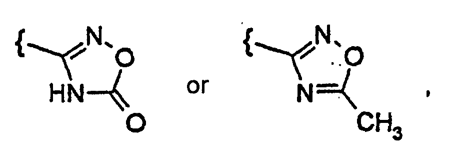 and R² and X are as defined in Claim 1,
or
d) for the preparation of compounds of the formula I
in which R¹ is 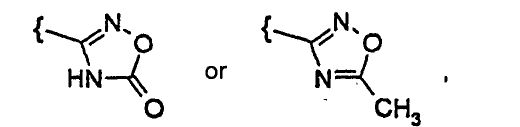
Y is -CONH-,
and R² and X are as defined in Claim 1,
a compound of the formula VI
R³-N=C=O VI
in which R³ is as defined in Claim 1,
is reacted with a compound of the formula V 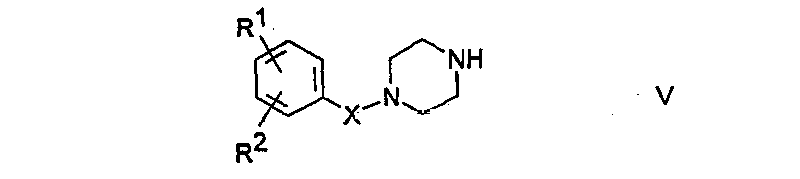 in which R¹ is 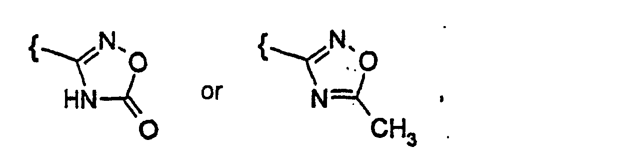 and R² and X are as defined in Claim 1,
or
e) for the preparation of compounds of the formula I
in which R¹ is -C(=NH)-NH₂,
a cyano group is converted into an amidino group,
f) and/or one or more radical(s) R¹, R² and/or R³ in a compound of the formula I is (are) converted into one or more radical(s) R¹, R² and/or R³
by, for example,
i) hydrolysing an ester group to a carboxyl group,
ii) reducing a nitro group,
iii) acylating an amino group,
g) and/or a base or acid of the formula I is converted into one of its salts.

4. Process for the preparation of pharmaceutical preparations, **characterised in that** a compound of the formula I according to Claim 1 and/or one of its physiologically acceptable salts is converted into a suitable dosage form together with at least one solid, liquid or semi-liquid excipient or adjuvant.

5. Pharmaceutical preparation, **characterised by** a content of at least one compound of the formula I according to Claim 1 and/or one of its physiologically acceptable salts.

6. Compounds of the formula I according to Claim 1 and physiologically acceptable salts thereof for combating thromboses, myocardial infarction, arteriosclerosis, inflammation, apoplexia, angina pectoris, restenosis after angioplasty and claudicatio intermittens.

7. Medicament of the formula I according to Claim 1 and physiologically acceptable salts thereof as inhibitors of coagulation factor Xa.

8. Use of compounds of the formula I according to Claim 1 and/or physiologically acceptable salts thereof for the preparation of a medicament.

9. Use of compounds of the formula I according to Claim 1 and/or physiologically acceptable salts thereof for the preparation of a medicament for combating thromboses, myocardial infarction, arteriosclerosis, inflammation, apoplexia, angina pectoris, restenosis after angioplasty and claudicatio intermittens.

## Revendications

1. Composés de formule I 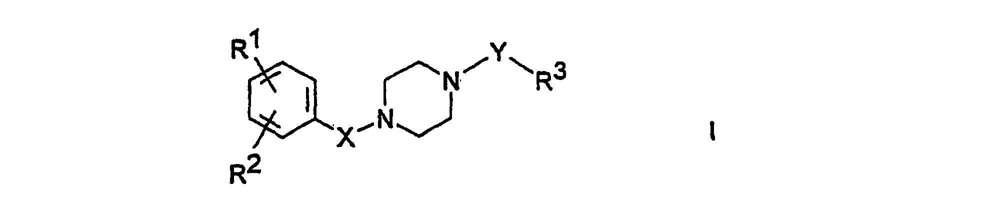 dans laquelle
R¹ représente -C(=NH)-NH₂, pouvant également être monosubstitué par -COA, -CO-[C(R⁶)₂]ₙ-Ar, -COOA, -OH ou par un groupement protecteur d'amine classique, ou 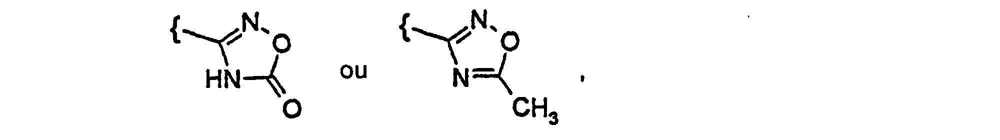
R² représente H, A, OR⁶, N(R⁶)₂, NO₂, CN, Hal, NHCOA, NHCOAr, NHSO₂A, NHSO₂Ar, COOR⁶, CON(R⁶)₂, CONHAr, COR⁶, COAr, S(O)ₙA ou S(O)ₙAr,
R³ représente A, cycloalkyle, -[C(R⁶)₂]Ar, -[C(R⁶)₂]ₙ-O-Ar, -[C(R⁶)₂]ₙHét ou -C(R⁶)₂=C(R⁶)₂-Ar,
R⁶ représente H, A ou benzyle,
X est absent ou représente -CO-, -C(R⁶)₂-, -C(R⁶)₂-C(R⁶)₂-, -C(R⁶)₂-CO-, -C(R⁶)₂-C(R⁶)₂-CO-, -C(R⁶)=C(R⁶)-CO-, NR⁶CO-, -N{[C(R⁶)₂]ₙ-COOR⁶}-CO- ou -C(COOR⁶)R⁶-C(R⁶)₂-CO-,
Y représente -C(R⁶)₂-, -SO₂-, -CO-, -COO- ou -CONR⁶-,
A représente alkyle ayant de 1 à 20 atomes de carbone, dans lequel un ou deux groupements CH₂ peut(peuvent) être remplacé(s) par des atomes de O ou de S ou par des groupements -CR⁶=CR⁶- et/ou de 1 à 7 atomes de H peuvent être remplacés par F,
Ar représente phényle ou naphtyle, chacun parmi ceux-ci étant non substitué ou monosubstitué, disubstitué ou trisubstitué par A, Ar', OR⁶, N(R⁶)₂, NO₂, CN, Hal, NHCOA, NHCOAr', NHSO₂A, NHSO₂Ar', COOR⁶, CON(R⁶)₂, CONHAr', COR⁶, COAr', S(O)ₙA ou S(O)ₙAr,
Ar' représente phényle ou naphtyle, chacun parmi ceux-ci étant non substitué ou monosubstitué, disubstitué ou trisubstitué par A, OR⁶, N(R⁶)₂, NO₂, CN, Hal, NHCOA, COOR⁶, CON(R⁶)₂, COR⁶ ou S(O)ₙA,
Hét représente un noyau hétérocyclique monocyclique ou bicyclique, saturé ou insaturé, qui possède un, deux, trois ou quatre hétéroatomes identiques ou différents, tels que l'azote, l'oxygène et le soufre, et qui est non substitué, ou monosubstitué ou polysubstitué par Hal, A, Ar', COOR⁶, CN, N(R⁶)₂, NO₂, Ar-CONH-CH₂ et/ou un oxygène de carbonyle,
Hal représente F, Cl, Br ou I,
n vaut 0, 1 ou 2,
et les sels de ceux-ci.

2. Composés selon la revendication 1,
a) la 4-[4-(4-propylphénylsulfonyl)-1-pipérazinylcarbonyl]benzamidine;
b) la 4-[4-(3-amino-4-chlorophénylsulfonyl)-1-pipérazinylcarbonyl]-benzamidine;
c) la 4-[4-(6-chloronaphthalène-2-sulfonyl)-1-pipérazinylcarbonyl]-benzamidine;
d) la 4-[4-(2-phénylvinylsulfonyl)-1-pipérazinylcarbonyl]benzamidine;
et les sels de ceux-ci.

3. Procédé de préparation de composés de formule I selon la revendication 1 et de sels de ceux-ci, **caractérisé en ce que**
a) ils sont libérés depuis l'un de leurs dérivés fonctionnels par un traitement par un agent de solvolyse ou d'hydrogénolyse par
i) la libération d'un groupement amidino depuis son dérivé oxadiazole par hydrogénolyse,
ii) le remplacement d'un groupement protecteur d'amine classique par l'hydrogène par un traitement par un agent de solvolyse ou d'hydrogénolyse ou
la libération d'un groupement amine protégé par un groupement protecteur classique,
ou
b) pour la préparation de composés de formule I
dans laquelle
R¹ représente 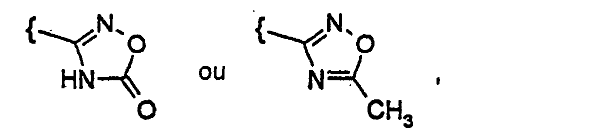
X représente -CO- ou -C(R⁶)₂-CO-,
et R², R³ et Y sont tels que définis selon la revendication 1,
un composé de formule II 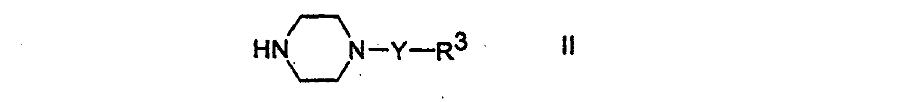 dans laquelle
R³ et Y sont tels que définis selon la revendication 1,
est réagi avec un composé de formule III 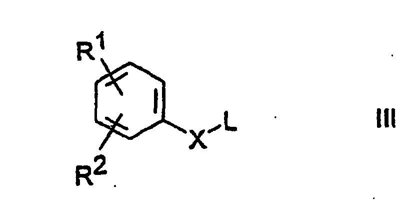 dans laquelle
R¹ représente 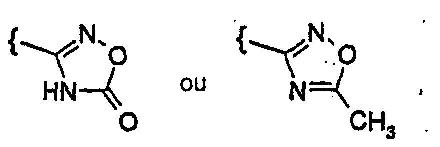
X représente -CO- ou -C(R⁶)₂-CO-,
R² est tel que défini selon la revendication 1,
et L représente Cl, Br, I ou un groupement OH libre ou modifié de manière réactive et fonctionnelle,
ou
c) pour la préparation de composés de formule I dans laquelle
R¹ représente 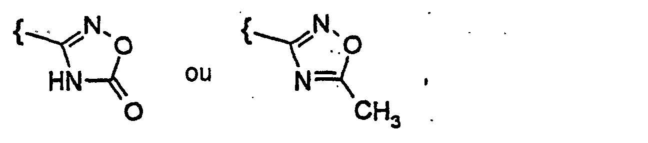
Y représente -SO₂-, -CO-, -COO- ou -C(R⁶)₂-,
et R² et X sont tels que définis selon la revendication 1,
un composé de formule IV
L-Y-R³ IV
dans laquelle
Y représente -SO₂-, -CO-, -COO- ou -C(R⁶)₂-,
R³ est tel que défini selon la revendication 1,
et L représente Cl, Br, I ou un groupement OH libre ou modifié de manière réactive et fonctionnelle,
est réagi avec un composé de formule V 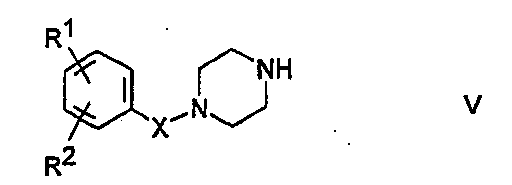 dans laquelle
R¹ représente 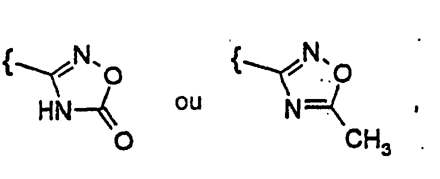
et R² et X sont tels que définis selon la revendication 1,
ou
d) pour la préparation de composés de formule I dans laquelle
R¹ représente 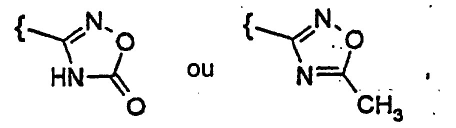
Y représente -CONH-,
et R² et X sont tels que définis selon la revendication 1,
un composé de formule VI
R³-N=C=O VI
dans laquelle R³ est tel que défini selon la revendication 1,
est réagi avec un composé de formule V 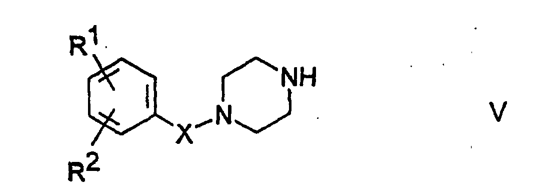 dans laquelle
R¹ représente 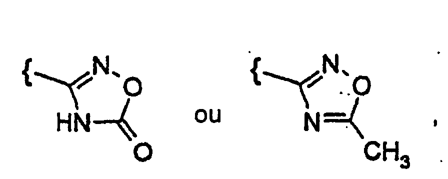
et R² et X sont tels que définis selon la revendication 1,
ou
e) pour la préparation de composés de formule I
dans laquelle R¹ représente -C(=NH)-NH₂,
un groupement cyano est converti en un groupement amidino,
f) et/ou un ou plusieurs radical(aux) R¹, R² et/ou R³ dans un composé de formule I est (sont) converti(s) en un ou plusieurs radical(aux) R¹, R² et/ou R³,
par exemple, par
i) l'hydrolyse d'un groupement ester en un groupement carboxyle,
ii) la réduction d'un groupement nitro,
iii) l'acylation d'un groupement amine,
g) et/ou une base ou un acide de formule I est converti(e) en l'un de ses sels.

4. Procédé de préparation de préparations pharmaceutiques, **caractérisé en ce qu'**un composé de formule I selon la revendication 1 et/ou l'un de ses sels physiologiquement acceptables est mis sous une forme de dosage convenable conjointement avec au moins un excipient ou adjuvant solide, liquide ou semi-liquide.

5. Préparation pharmaceutique, **caractérisée par** une teneur en au moins un composé de formule I selon la revendication 1 et/ou l'un de ses sels physiologiquement acceptables.

6. Composés de formule I selon la revendication 1 et les sels physiologiquement acceptables de ceux-ci pour lutter contre les thromboses, I'infarctus du myocarde, l'artériosclérose, les inflammations, l'apoplexie, l'angine de poitrine, la resténose post-angioplastie et la boiterie intermittente.

7. Médicament de formule I selon la revendication 1 et les sels physiologiquement acceptables de celui-ci en tant qu'inhibiteurs du facteur de coagulation Xa.

8. Utilisation de composés de formule I selon la revendication 1 et/ou des sels physiologiquement acceptables de ceux-ci pour la préparation d'un médicament.

9. Utilisation de composés de formule I selon la revendication 1 et/ou des sels physiologiquement acceptables de ceux-ci pour la préparation d'un médicament pour lutter contre les thromboses, l'infarctus du myocarde, l'artériosclérose, les inflammations, l'apoplexie, l'angine de poitrine, la resténose post-angioplastie et la boiterie intermittente.
